# EUROPEAN PATENT APPLICATION

(11) **EP 3 569 249 A1**
(43) Date of publication of application: **20.11.2019**
(21) Application number: 17885962.5
(22) Date of filing: 27.12.2017
(51) Int. Cl.: A61K 45/06, A61K 9/14, A61K 9/50, A61K 9/70, A61K 31/24, A61K 31/366, A61K 31/4174, A61K 31/4418, A61K 31/4709, A61K 31/496, A61K 31/519, A61K 31/522, A61L 15/40, A61L 15/64, A61P 9/00, A61P 9/04, A61P 9/06, A61P 9/10, A61P 43/00, C07D 209/34, C07D 213/64

(54) **MEDICINAL COMPOSITION FOR TREATING INTRACTABLE HEART DISEASE**

(30) Priority: 27.12.2016 JP 2016254279
(71) Applicant: Osaka University, Suita-shi, Osaka 565-0871 (JP); Cardio Incorporated, Kobe-shi Hyogo 650-0047 (JP)
(72) Inventor: SAWA, Yoshiki, Suita-shi Osaka 565-0871 (JP); MIYAGAWA, Shigeru, Suita-shi Osaka 565-0871 (JP); SAKAI, Yoshiki, Suita-shi Osaka 565-0871 (JP); YANAGI, Yasuhiro, Kobe-shi Hyogo 658-0063 (JP)
(74) Representative: Long, Giorgio
(86) International application number: PCT/JP2017/047100
(87) International publication number: WO 2018/124236

(57) **Abstract**

The present invention provides a pharmaceutical composition for use in treating an intractable heart tissue fibrosis disease accompanied by chronic heart failure. The pharmaceutical composition for use in treating an intractable heart tissue fibrosis disease accompanied by chronic heart failure comprises, as an active ingredient, at least one member selected from the group consisting of protease inhibitors, thromboxane A₂ synthase inhibitors, thromboxane A₂ synthase antagonists, phosphodiesterase (PDE) inhibitors, tyrosine kinase inhibitors, HMG-CoA reductase inhibitors, and antifibrotic agents. (The pharmaceutical composition includes biodegradable polymer-encapsulated, long-acting preparations thereof.)

## Description

### Technical Field

The present invention relates to a pharmaceutical composition for use in treating an intractable heart disease.

### Background Art

Patients with dilated cardiomyopathy (DCM), a disease for which orphans drugs have been designated, are considered to have a one-year mortality rate of 75% during end-stage heart failure. A definitive therapy for DCM is heart transplant. However, the unequivocal shortage of donors remains unchanged, even after revision of the Organ Transplantation Act. As pharmacotherapy, β blockers, angiotensin II receptor blockers (ARB), ACE inhibitors, diuretics, digitalis, anti-aldosterone drugs, oral cardiotonic agents, etc., are widely used; however, their effects are insufficient. Patients for whom heart transplant is suitable have a left ventricular assist device (LVAD) implanted as a palliative treatment. However, LVAD implantation has a high risk of complications (cerebral infarction, infectious diseases, etc.), and many patients die while waiting for heart transplant. In view of the severe donor shortage in Japan, it is an urgent task to discover and develop a new regenerative medicine whose purpose, through early treatment intervention, is to avoid or delay heart transplant or LVAD implantation.

Myocardial infarction associated with coronary arteriosclerosis is one of the three major diseases in Japan. In Japan, which faces a globally unprecedented aging society, the number of patients suffering from myocardial infarction is expected to further increase in the future. The "Patient Survey" of fiscal year 2011 by the Ministry of Health, Labour and Welfare reported 756,000 cases of ischemic heart disease, 558,000 cases of angina pectoris, 41,000 cases of acute myocardial infarction, and 110,000 cases of old myocardial infarction. Although no specific number of ischemic cardiomyopathy cases is indicated, ischemic cardiomyopathy is considered to account for about 10 to 20% of old myocardial infarction.

Ischemic cardiomyopathy is a progressive, intractable, frequently occurring disease with a poor prognosis, and is expected to increase in the future. Non-invasive treatments, in which pharmacotherapy is a main therapy, and revascularization are currently performed as standard treatments. However, neither pharmacotherapy nor revascularization is considered to be a definitive treatment. The development of a novel treatment by an approach different from that of previous treatments is desired.

Ischemic cardiomyopathy is a disease state in which a wide range of myocardial ischemia or myocardial infarction caused by coronary arteriosclerotic disease severely reduces left ventricular wall motion and chronic congestive heart failure develops. "Guidelines for Device Therapy: Implantable Left Ventricular Assist Device for Patients with Severe Heart Failure (2013)" defines ischemic cardiomyopathy as "a wide range of myocardial infarction or multi-branched lesion case with abnormal wall motion and significantly reduced cardiac function." Since systolic and diastolic functions of the left ventricle are reduced at sites of myocardial ischemia or myocardial infarction, decreased cardiac output and increased left ventricular end-diastolic pressure cause lung congestion. Further, compensatory left ventricular enlargement occurs, and progress of the enlargement leads to a progressive expansion of the left ventricle called left ventricular remodeling, and hypofunction.

The standard treatment method for ischemic cardiomyopathy is performed by a combination of noninvasive treatment and invasive treatment. Noninvasive treatment mainly consists of pharmacotherapy. β blockers, angiotensin II receptor blockers (ARB), angiotensin-converting enzyme (ACE) inhibitors, antiplatelet drugs, nitrate drugs, and Ca antagonists are widely used; and cardiac rehabilitation, adaptive support ventilation (ASV), etc., are also used in combination. On the other hand, invasive treatment mainly consists of coronary artery bypass surgery and percutaneous transluminal coronary angioplasty. However, these are only applicable to cases having high-grade central stenosis or obstructive lesion, and a sufficient myocardial perfusion territory on its peripheral side.

Although noninvasive treatments are performed in all cases of ischemic cardiomyopathy, noninvasive treatments alone are reported to achieve a 5-year of about 50% in patients having a left ventricular ejection fraction of 40% or less, as determined by a cardiac ultrasound examination. The 5-year survival rate of patients additionally subjected to invasive treatments improves to 60-70%; however, their prognosis is still considered to be poor.

Although there are age limitations and disease complications, cases in which lesion progression is observed and end-stage symptoms are presented despite fully performed treatments are suitable for heart transplant and implantable left ventricular assist devices (LVAD).

The present inventors have already developed a therapeutic method for applying an autologous skeletal muscle-derived myoblast cell sheet to the heart of a patient with severe cardiomyopathy (heart sheet) (Non-Patent Literature (NPL) 1). The cell sheet was approved as a "regenerative medical product" in Japan in 2015. The present inventors further clarified that this therapeutic method provides angiogenesis and myocardial regeneration effects by various *in vivo* regeneration factors (HGF, VEGF, SDF-1, etc.) secreted from the cell sheet (Non-Patent Document 2).

Further, the present inventors newly discovered ONO-1301 ((E)-[5-[2-[1-phenyl-1-(3-pyridyl)methylideneaminooxy]ethyl]-7,8-dihydronaphthalen-1-yloxy]acetic acid) as a low-molecular synthetic compound that has the mechanism of angiogenesis/myocardial regeneration action. More specifically, ONO-1301, an oxime derivative, was originally discovered as a prostaglandin (PG) I2 receptor agonist, and developed as an oral antithrombotic agent (Patent Literature (PTL) 1). However, its development was discontinued due to the narrow trade-off between side effects (e.g., vasodilating action, diarrhea, etc.) and efficacy (platelet aggregation-inhibitory action).

Since prostaglandin (PG) 2 receptor (IP) agonists, such as ONO-1301, beraprost, and selexipag (NS-304); EP2 agonists; and EP4 agonists act on fibroblasts etc. at a concentration lower than the concentration at which platelet aggregation-inhibitory action is exhibited, and promote production of many various in vivo regeneration factors, such as a hepatocyte growth factor (HGF), vascular endothelial cell growth factor (VEGF), stromal cell-derived factor (SDF-1), high mobility group box protein 1 (HMGB1), fibroblast growth factor (a/bFGF), epidermal growth factor (EGF), hypoxia induced factor (HIF), and granulocyte colony-stimulating factor (G-CSF), the present inventors found new indications for these drugs as regenerative medicine, such as intractable heart diseases (Patent Literature (PTL) 2). This implies that PGI 2 and PGE 2 are involved in the early stage of a wound-healing process associated with inflammation, ischemia, etc. More specifically, it is suggested that since cyclooxygenase (COX II) is induced and PGs (e.g., PGI2 and PGE2) are biosynthesized at an ischemia and/or inflammation site, many *in vivo* regeneration factors are induced, and wounds are healed.

The present inventors further developed a sustained-release microsphere preparation (YS-1402) comprising ONO-1301 encapsulated in a biodegradable polymer (poly(lactic-co-glycolic acid); PLGA), and newly established a cardiac patch application method for administering this preparation (Patent Literature (PTL) 3 and Patent Literature (PTL) 4).

An *in vivo* induced heart regeneration therapy, which comprises applying to the heart a gelatin sheet impregnated with YS-1402 for administration to treat ischemic cardiomyopathy and dilated cardiomyopathy, is currently the subject of an investigator-initiated clinical trial (P-IIIa test) (Non-Patent Literature (NPL) 3). The present inventors are also developing a disease site-specific liposome nanosphere preparation comprising ONO-1301 or the like. Further, the present inventors plan to perform a clinical trial for a therapeutic method comprising applying an iPS cardiomyocyte sheet to the heart. Although these cell therapies and cardiac patch application methods can be expected to have selective effects due to their topical administration to the heart, these methods have many problems in terms of invasiveness, economy, safety, versatility, etc.

On the other hand, a lung disease site-specific therapeutic agent (PTL 5) is known, whose mechanism is such that intravenous injection of a small amount of a YS-1402 preparation accumulates the YS-1402 preparation in the lungs and allows gradual release of a pharmaceutical agent in the lungs, thereby maintaining a high concentration of the pharmaceutical agent in the lungs (Patent Literature (PTL) 5). However, this method has a risk such that mass administration may cause the development of pulmonary embolism, and thus has a safety problem.

### Citation List

### Patent Literature

PTL 1: Patent No. 2691679
PTL 2: WO2004/032965
PTL 3: WO2008/047863
PTL 4: WO2014/046065
PTL 5: WO2014/069401

### Non-patent Literature

NPL 1: Surg. Today (2012) 42:181-184
NPL 2: Ann. Thorac. Surg. (2011) 91:320-9
NPL 3: Heart Fail Rev. (2015) 20:401-413

### Summary of Invention

### Technical Problem

The present inventors investigated a versatile, economical, safe, and minimally invasive cardiovascular/myocardial regeneration therapeutic agent, which can be used in place of LVAD implantation, heart transplant, cell sheet therapy requiring open chest surgery, or the like; and which enables early therapeutic intervention by a non-invasive versatile administration method, such as oral administration or intermittent subcutaneous administration, to inhibit aggravation of intractable heart disease, and delay and avoid LVAD implantation and heart transplant. As a result, the inventors surprisingly found that some compounds of already-commercially available pharmaceutical products are effective against intractable heart diseases when administered at a safe dose.

While setting research themes, such as "pathological analysis of metabolome and/or proteasome," "development of drug discovery screening system using disease-specific iPS cells," and "research on regenerative drug discovery using iPS cell-derived cardiomyocytes in cardiovascular diseases," for intractable heart disease patients, the present inventors screened for search of therapeutic drugs for intractable heart diseases in an *in vitro* system using vascular endothelial cells, fibroblasts, iPS cardiomyocytes, etc.

The inventors performed *in vivo* screening mainly by using several pharmaceutical products selected by such *in vitro* screening, from the viewpoint of drug repositioning; and using, for example, spontaneous dilated cardiomyopathy hamster models, rat coronary artery ischemia models, and canine rapid pacing dilated cardiomyopathy models. As a result, the inventors confirmed that some pharmaceutical products have effects at a safe dose.
These selected pharmaceutical products are ground-breaking in that the pharmaceutical products do not have an antihypertensive action, and can therefore be administered concomitantly with β blockers, angiotensin-converting enzyme (ACE) inhibitors, angiotensin II receptor blocker (ARB), or the like, which are currently used as drugs for heart failure having antihypertensive action.

The selected mechanism of action includes protease inhibitors, thromboxane A₂ synthase inhibitors, antagonists of thromboxane A₂ synthase inhibitors, phosphodiesterase (PDE) inhibitors, kinase inhibitors, HMG-CoA reductase inhibitors, antifibrotic agents, and the like. Examples of PDE inhibitors include nonselective PDE inhibitors, PDEIII inhibitors, PDEV inhibitors, and the like. Examples of kinase inhibitors include tyrosine kinase inhibitors.

Further, the present inventors have newly prepared microsphere preparations comprising these selected pharmaceutical agents encapsulated in a biodegradable polymer. When these preparations are subcutaneously or intramuscularly administered about once a week to every three months, they can stably exhibit intravenous infusion-like blood kinetics over a long period of time. Since these preparations are more convenient in intermittent administration as compared with daily oral administration, and exhibit intravenous infusion-like blood kinetics, side effects can be expected to be avoided due to the avoidance of high blood concentrations; and prolonged effects can be expected due to long-term sustained blood kinetics.

Accordingly, an object of the present invention is to provide a pharmaceutical composition that is effective for treating an intractable heart tissue fibrosis disease, wherein the intractable heart tissue fibrosis disease accompanied by chronic heart failure to be treated is dilated cardiomyopathy, ischemic cardiomyopathy, myocardial infarction, angina pectoris, arteriosclerosis, vasculitis syndrome, myocarditis, hypertrophic cardiomyopathy, aortic valve stenosis, valvular disease, aortic regurgitation, HFpEF (heart failure with preserved ejection fraction), diastolic dysfunction, systolic dysfunction, supraventricular tachyarrhythmia, congestive heart failure, coronary artery disease, idiopathic cardiomyopathy, and atrial fibrillation.

More specifically, an object of the present invention is to provide a versatile pharmaceutical product that can be administered in combination with antihypertensive agents currently used as chronic heart failure drugs, such as ACE inhibitors, ARB, and β blockers; and thereby provide a novel, ground-breaking therapeutic agent for heart tissue fibrosis diseases for the purpose of delaying and avoiding left ventricular assist device implantation and heart transplant by noninvasively administering, as an early therapeutic intervention, a selected existing pharmaceutical product that does not exhibit antihypertensive action.

### Solution to Problem

The present inventors selected about 2,000 kinds from about 16,000 kinds of pharmaceutical products commercially available in Japan, and extensively evaluated them through pathological analysis for cardiac diseases and *in vitro* systems using iPS cells or the like. As a result, several pharmaceutical products were found to be effective for heart diseases because of their angiogenic action, anti-fibrotic, anti-apoptotic action, cytoprotective action, reverse remodeling action, mesenchymal stem cell differentiation/induction action, *in vivo* regeneration factor induction action, anti-inflammatory action, circulation-improving action, and the like. With these actions being used as characteristics, search screening for heart disease therapeutic drugs was performed *in vitro.* Several pharmaceutical products selected by such *in vitro* screening for drug repositioning (drug repurposing) were mainly subjected to *in vivo* screening using spontaneous dilated cardiomyopathy hamster models, rat coronary artery ischemia models, canine rapid pacing dilated cardiomyopathy models, etc. The doses for these disease models were evaluated *in vivo* by repeated oral administration, based on the no-observed-adverse-effect level (NOAEL) in a long-term toxicity study and clinical dose. The pharmaceutical products whose efficacy was newly found are (1) protease inhibitors, (2) phosphodiesterase (PDE) inhibitors, (3) tyrosine kinase inhibitors, (4) thromboxane (TX) A₂ synthase inhibitors, (5) HMG-CoA reductase inhibitors, and (6) antifibrotic agents. Prostaglandin IP receptor agonists also similarly demonstrated efficacy, but these agonists are known pharmaceutical agents whose efficacy was already found by the present inventors (Patent Literature (PTL) 2).

It is ground-breaking that microsphere (MS) preparations, which comprise at least one of these groups of found drugs encapsulated in a biodegradable polymer, poly(lactic-co-glycolic acid) (PLGA), exhibit efficacy, when administered by intermittent subcutaneous injection or intramuscular injection in an amount that is not more than one-tenth of the total oral dose amount, about once every two weeks to three months.

The present inventors conducted extensive research. As a result, surprisingly, the present inventors newly found that pharmaceutical products having the above 6 types of mechanisms of actions can achieve the above object. The inventors further found that these biodegradable encapsulated, long-acting microsphere preparations are also useful in intermittent administration, and accomplished the present invention.

More specifically, the present invention includes the following embodiments.
[1] A pharmaceutical composition for use in preventing and/or treating an intractable heart tissue fibrosis disease accompanied by chronic heart failure.
[2] The pharmaceutical composition according to Item [1], comprising a protease inhibitor.
[3] The pharmaceutical composition according to Item [1], comprising a thromboxane A₂ synthase inhibitor and/or a thromboxane A₂ synthase antagonist.
[4] The pharmaceutical composition according to Item [1], comprising a phosphodiesterase (PDE) inhibitor.
[5] The pharmaceutical composition according to Item [1], comprising a tyrosine kinase inhibitor.
[6] The pharmaceutical composition according to Item [1], comprising an HMG-CoA reductase inhibitor.
[7] The pharmaceutical composition according to Item [1], comprising an antifibrotic agent.
[8] The pharmaceutical composition according to Item [1], comprising at least two members selected from the group consisting of a protease inhibitor, a thromboxane A₂ synthase inhibitor, a thromboxane A₂ synthase antagonist, a phosphodiesterase (PDE) inhibitor, a tyrosine kinase inhibitor, an HMG-CoA reductase inhibitor, and an antifibrotic agent.
[9] The pharmaceutical composition according to any one of Items [1] to [8], comprising at least one member selected from the group consisting of the following compounds (1) to (6) and salts thereof:
   (1) camostat as a protease inhibitor;
   (2) ozagrel as a thromboxane A₂ synthase inhibitor;
   (3) theophylline, cilostazol, and sildenafil as phosphodiesterase inhibitors;
   (4) nintedanib as a tyrosine kinase inhibitor;
   (5) lovastatin as an HMG-CoA reductase inhibitor; and
   (6) pirfenidone as an antifibrotic agent.
[10] The pharmaceutical composition according to any one of Items [1] to [9], which is a long-acting preparation further comprising a biodegradable polymer.
[11] The pharmaceutical composition according to Item [10], wherein the long-acting preparation is a microsphere preparation, a microcapsule preparation, or a nanosphere preparation.
[12] The pharmaceutical composition according to Item [10], wherein the biodegradable polymer is a poly(lactic-co-glycolic acid), and the long-acting preparation is a microsphere preparation.
[13] The pharmaceutical composition according to Item [11], which comprises at least one member selected from the group consisting of the following compounds (1) to (5) and salts thereof:
   (1) camostat as a protease inhibitor;
   (2) ozagrel as a thromboxane A₂ synthase inhibitor;
   (3) cilostazol and sildenafil as phosphodiesterase inhibitors;
   (4) nintedanib as a tyrosine kinase inhibitor; and
   (5) pirfenidone as an antifibrotic agent.
[14] The pharmaceutical composition according to any one of [1] to [13], which is for oral administration, intravenous administration, intracoronary administration, inhalation, intramuscular injection, subcutaneous administration, transmucosal administration, transdermal administration, or cardiac patch application.
[15] The pharmaceutical composition according to any one of [1] to [14], wherein the intractable heart tissue fibrosis disease accompanied by chronic heart failure is dilated cardiomyopathy, ischemic cardiomyopathy, myocardial infarction, angina pectoris, arteriosclerosis, vasculitis syndrome, myocarditis, hypertrophic cardiomyopathy, aortic valve stenosis, valvular disease, aortic regurgitation, HFpEF (heart failure with preserved ejection fraction), diastolic dysfunction, contractile dysfunction, supraventricular tachyarrhythmia, congestive heart failure, coronary artery disease, idiopathic cardiomyopathy, or atrial fibrillation.

### Advantageous Effects of Invention

Groups of compounds represented by pharmaceutical products having the 6 types of mechanisms of actions may be commercially available pharmaceutical products of these types; or may be new compounds that will be developed in the future, and that have such mechanisms of actions.

Pharmaceutical preparations comprising these compounds may be commercially available pharmaceutical preparations, or new pharmaceutical preparations. Examples of new pharmaceutical preparations include improved oral preparations, combination drugs, biodegradable polymer-encapsulated, sustained release microsphere preparations obtained by various methods, nanosphere preparations, and the like. The pharmaceutical agents of the present invention including these various pharmaceutical preparations can be administered, for example, orally, intravenously, intra-arterially, intramuscularly, subcutaneously, by inhalation, by patch application administration, or in the form of an ointment. Basically, the pharmaceutical agents of the present invention are improved oral preparations comprising these compounds with excellent administration compliance, commercially available preparations, or sustained-release preparations comprising these compounds encapsulated in a biodegradable polymer; and are used for subcutaneous administration, intramuscular injection, organ patch application, intravenous injection, or inhalation administration.

The present inventors have already reported in detail the efficacy of prostaglandin IP receptor agonists against heart diseases in WO2004/032965, i.e., Patent Literature (PLT) 2. PTL 2 further discloses that EP4 receptor agonists, PGI2 derivatives, PGE1 derivatives, and PGE2 derivatives, as well as IP receptor agonists, are effective against prostaglandin.

In principle, these 6 types of pharmaceutical products (including biodegradable polymer-encapsulated, sustained-release preparations) are preferably administered in combination with a therapeutic drug currently used as an antihypertensive agent, such as a β blocker, ARB, or ACE inhibitor, or as a diuretic agent, etc. These 6 types of pharmaceutical products can be preferably used singly or in combination of two or more as long as the efficacy can be expected, and side effects do not occur. In view of medication convenience and effect-increasing action, a combination drug comprising two or three or more pharmaceutical products having at least one of 6 types of mechanisms of actions, in addition to a currently used antihypertensive agent, can be prepared and used.

### Brief Description of Drawings

Fig. 1 is a graph showing changes in the survival rate in long-term administration.
Fig. 2 is a graph of survival rate curves (Control: no administration, ONO-1301: oral administration (once per 2 days, for 26 weeks), *: P < 0.05, significant difference (between the Control and a Group receiving ONO-1301 at 3 mg/kg (Log rank test)).
Fig. 3 is a graph showing changes in body weight (Δ: time after the start of administration, Control: no administration; ONO-1301: oral administration (once per 2 days, for 26 weeks); each value indicates the mean±S.D.; **: significant difference compared to the Control, P < 0.01 (Student's t-test)).
Fig. 4 is a graph showing changes in cardiac function (EF) (changes from the amount at the time of grouping; Δ) (Control: no administration; ONO-1301: oral administration (once per 2 days, 26 weeks); each value indicates the mean±S.D.; **: significant difference compared to Control, P < 0.05 (Student's *t*-test)).
Fig. 5 is an optical microscope photograph of ozagrel hydrochloride.
Fig. 6 is a UV absorption spectrum of ozagrel hydrochloride.
Fig. 7 is an optical micrograph of camostat mesylate.
Fig. 8 is a UV absorption spectrum of camostat mesylate.
Fig. 9 is an optical microscope photograph of sildenafil citrate.
Fig. 10 is a UV absorption spectrum of sildenafil citrate.

### Description of Embodiments

### 1. Protease Inhibitor

Proteases mainly include serine protease, cysteine protease, metalloprotease, aspartic protease, and the like.

As a protease inhibitor, camostat mesilate is used in the form of an oral preparation for remission of acute symptoms in chronic pancreatitis, and postoperative reflux esophagitis. Camostat exhibits strong inhibitory effects on trypsin, plasma kallikrein, plasmin, thrombin, prostasin, and C1r-, C1 esterase. On the other hand, camostat exhibits weak inhibitory effects on pancreatin and pancreatic kallikrein; and exhibits no inhibitory effects on α-chymotrypsin, pepsin, bromelain, serratiopeptidase, or elastase 5 (*in vitro*).

As an injectable preparation, gabexate mesilate is used for various diseases (acute pancreatitis, acute exacerbation phase of chronic recurrent pancreatitis, postoperative acute pancreatitis) accompanied by deviation of proteases (e.g., trypsin, kallikrein, plasmin), and disseminated intravascular coagulation (DIC). Gabexate mesilate inhibits trypsin, kallikrein (kinin system), thrombin (coagulation system), activated factor X (coagulation system), plasmin (fibrinolytic system), C1-esterase (complement system), and the like (*in vitro*).

Nafamostat mesilate is used to ameliorate acute symptoms of pancreatitis, and prevent perfusion blood coagulation during blood extracorporeal circulation in patients with disseminated intravascular coagulation (DIC) and hemorrhagic lesions or bleeding tendency. Nafamostat mesilate has potent inhibitory effects on the blood coagulation-fibrinolysis system (thrombin, XIIa, Xa, VIIa, plasmin), kallikrein-kinin system (kallikrein), complement system (C1r, C1s, B, D), and pancreatic enzymes (trypsin, pancreatic kallikrein) (*in vitro*).

Sivelestat sodium hydrate is used as a neutrophil elastase inhibitor, and as an ameliorating agent for acute lung injury accompanied by systemic inflammatory response syndrome (SIRS).

Various investigations revealed that serine protease inhibitors and elastase inhibitors are effective against heart diseases; and that plasmin inhibitors, plasma kallikrein inhibitors, thrombin inhibitors, prostasin inhibitors, and elastase inhibitors are particularly effective.

The inhibitors may be selective for one of these target proteases, or may have inhibitory effects on two or more proteases; both are efficacious. To complete the present invention, investigation was performed using camostat mesilate, which mainly inhibit serine protease, as a representative (see the Examples). However, previously known protease inhibitors, or protease inhibitors that will be developed in the future, are also usable.

The biodegradable polymer-encapsulated, sustained-release preparations of these inhibitors are also efficacious. Poly(lactic-co-glycolic acid) (PLGA) microsphere (MS) preparations of camostat mesylate and sivelestat sodium hydrate are particularly effective.

### 2. Phosphodiesterase (PDE) Inhibitor

Phosphodiesterase (PDE) is an enzyme that regulates intracellular signal transduction by hydrolyzing intracellular second messengers, cAMP and cGMP, to 5'-AMP and 5'-GMP, respectively. For PDE, 21 kinds of genes have been cloned so far, and these are classified into 11 families (PDE 1 to PDE 11) according to differences in amino acid sequence homology, biochemical characteristics, and sensitivity to inhibitors.

Theophylline and aminophylline are used as nonselective PDE inhibitors against bronchial asthma, asthmatic (asthma-like) bronchitis, chronic bronchitis, and emphysema.

Cilostazol is a PDE III inhibitor, and is used as an oral preparation to ameliorate ischemic symptoms, such as ulcer, pain, and cold sensation based on chronic arterial occlusion; and as a recurrence inhibitor after the onset of cerebral infarction (except for cardiogenic embolism). On the other hand, amrinone, milrinone, and olprinone hydrochloride hydrate are also selective PDE III inhibitors, and are used as injectable formulations for acute heart failure.

Sildenafil citrate is a PDE V inhibitor, and has been used as an erectile dysfunction medication. Further, PDE IV inhibitors have been studied as atopic dermatitis and chronic obstructive pulmonary disease (COPD) drugs.

In the present invention, evaluation was performed by representatively using theophylline as a nonselective PDE inhibitor, cilostazol as a PDE III inhibitor, and sildenafil citrate as a PDE V inhibitor, respectively (see the Examples). These compounds are also used as acute heart failure or cardiotonic agents, but have never been used as heart tissue fibrosis disease therapeutic agents, such as dilated cardiomyopathy therapeutic agents.

The PDE inhibitor may be a known inhibitor, or a PDE inhibitor that will be developed in the future.

Furthermore, biodegradable polymer-encapsulated, sustained-release preparations of these compounds are also efficacious. Poly(lactic-co-glycolic acid)(PLGA) microsphere (MS) preparations of sildenafil citrate, theophylline, and cilostazol are particularly efficacious.

### 3. Tyrosine Kinase Inhibitor

Tyrosine kinase-type receptors are receptors for growth factors. Phosphorylation of tyrosine, among amino acids that can be phosphorylated (serine, threonine, tyrosine), promotes signal transduction and proliferate cells. Inhibiting this signaling system can suppress cell proliferation. There are many types of tyrosine kinase enzymes, and each enzyme has a role in changing tyrosine protein (autophosphorylation). Tyrosine kinase is abnormally activated in many cancers; and the protein altered thereby, tyrosine, is considered to bind to a signal transducer in cells, thus causing cell proliferation, invasion, metastasis, angiogenesis, etc. Accordingly, many tyrosine kinase inhibitors have been developed as anti-cancer agents.

For example, gefitinib (non-small cell lung cancer), erlotinib hydrochloride (lung cancer, pancreatic cancer), afatinib maleate (non-small cell lung cancer), and osimertinib (non-small cell lung cancer) are known. On the other hand, nintedanib ethanesulfonate is used for idiopathic pulmonary fibrosis.

To complete the present invention, evaluation was performed using nintedanib ethanesulfonate as a representative (see the Examples). However, known tyrosine kinase inhibitors, or tyrosine kinase inhibitors that will be developed in the future, are also usable.

Further, biodegradable polymer-encapsulated, sustained-release preparations of these compounds are also efficacious. Poly(lactic-co-glycolic acid) (PLGA) microsphere (MS) preparations of nintedanib ethanesulfonate are particularly efficacious.

### 4. TXA₂ Synthase Inhibitor and TXA₂ Receptor Antagonist

Arachidonic acid liberated from cell membrane phospholipid is oxidized to prostaglandin (PG) G2 with cyclooxygenase (COX) following the cascade of arachidonic acid, and further converted to PGH2 by peroxidase activity. PGH2 that then migrates to the cytoplasm thereafter is metabolized to various prostaglandins (PG) and thromboxane (TX) A₂ by various enzymes, and shows various physiological activities. TXA₂ is mainly produced in platelets, and is a substance that brings about platelet aggregation action, vascular permeability enhancement action, and vascular wall contraction. Further, TXA₂ has an action opposite to that of PGI2 produced mainly in vascular endothelial cells. PGI2 has, for example, platelet aggregation inhibitory action and vasodilator action; and homeostasis is maintained by the balance between TXA₂ and PGI2.

Further, biodegradable polymer-encapsulated, sustained-release preparations of these compounds are also efficacious. Poly(lactic-co-glycolic acid) (PLGA) microsphere (MS) preparations of ozagrel hydrochloride, ozagrel sodium, and seratrodast are particularly efficacious.

Ozagrel hydrochloride (oral preparation), which inhibits TXA₂ synthase and suppresses TXA₂ production, is used as a bronchial asthma drug. Ozagrel sodium (intravenous injection) is currently used as ameliorating agents for ameliorating cerebrovascular spasm after subarachnoid hemorrhage surgery, cerebral ischemic symptoms associated with cerebrovascular spasm, and movement disorders associated with cerebral thrombosis (acute phase). Further, seratrodast and ramatroban antagonistically inhibit thromboxane A₂ receptors, and are used as bronchial asthma or allergic rhinitis drugs. To complete the present invention, the present inventors performed evaluation using ozagrel hydrochloride as a representative (see the Examples). However, known inhibitors or antagonists, or inhibitors or antagonists that will be developed in the future, are also usable.

### 5. HMG-CoA Reductase Inhibitor

The HMG-CoA reductase inhibitor inhibits the action of HMG-CoA reductase, a mevalonate pathway rate-limiting enzyme, and thereby reduces liver cholesterol biosynthesis. As a result, LDL receptor expression in the liver is increased to maintain cholesterol homeostasis, and uptake of LDL cholesterol from the blood into the liver is promoted. LDL forms atheroma on blood vessel walls, and causes arteriosclerosis. Since continuous inhibition of cholesterol biosynthesis also reduces VLDL secretion into the blood, the plasma triglyceride level also decreases.

Examples of HMG-CoA reductase inhibitors include rosuvastatin, pitavastatin, atorvastatin, cerivastatin, fluvastatin, simvastatin, pravastatin, lovastatin, and the like, which are used as hypercholesterolemia and familial hypercholesterolemia drugs.

To complete the present invention, evaluation was performed by using lovastatin as a representative (see the Examples). However, known HMG-CoA reductase inhibitors or HMG-CoA reductase inhibitors that will be developed in the future are also usable.

Further, biodegradable polymer-encapsulated, sustained-release preparations of these compounds are also efficacious. Poly(lactic-co-glycolic acid) (PLGA) microsphere (MS) preparations of atorvastatin, pravastatin, fluvastatin, and lovastatin are particularly efficacious.

### 6. Antifibrotic agent

Pirfenidone is commercially available as a drug for idiopathic pulmonary fibrosis. Pirfenidone has been revealed to exhibit various pharmacological actions, such as anti-inflammatory action and antioxidant action, as well as anti-fibrotic action. However, the mechanisms of actions still remain unclear in many respects. An investigation using mouse bleomycin-induced pulmonary fibrosis models revealed that pirfenidone inhibits the production of growth factors, such as TGF-β (Transforming Growth Factor-β) involved in fibrotic formation; and also inhibits the decrease of interferon (IFN)-γ associated with the progression of pulmonary fibrosis. Such combined involvement of various actions is considered to lead to anti-fibrotic action.

To complete the present invention, evaluation was performed using pirferidone as a representative (see the Examples). However, known antifibrotic agents, or antifibrotic agents that will be developed in the future, are also usable.

Examples of antifibrotic agents associated with *in vivo* regeneration factor induction include AT1 receptor antagonists (ARB), peroxisome proliferator-activated receptor gamma (PPARγ) agonists, IL-1, TNF-α, INF, etc., in addition to cholera toxin (Cholera toxin), 8-bromo-cAMP, dibutyryl-cAMP, and forskolin.

Further, biodegradable polymer-encapsulated, sustained-release preparations of these antifibrotic agents are also efficacious. Poly(lactic-co-glycolic acid) (PLGA) microsphere (MS) preparations of atorvastatin, pravastatin, fluvastatin, and lovastatin are particularly efficacious.

### 7. Method for Producing the Compound According to the Present Invention

The compounds according to the present invention are already used as pharmaceutical products, and generally commercially available as reagents or pharmaceutical products. Biodegradable polymer-encapsulated, sustained-release preparations of these compounds can be produced by known methods (WO2004/032965).

### 8. Subject Receiving the Pharmaceutical Composition of the Present Invention

Preferable examples of subjects receiving the pharmaceutical composition of the present invention include mammals having a target intractable heart tissue fibrosis disease accompanied by chronic heart failure, wherein the target disease is dilated cardiomyopathy, ischemic cardiomyopathy, myocardial infarction, angina pectoris, arteriosclerosis, vasculitis syndrome, myocarditis, hypertrophic cardiomyopathy, aortic valve stenosis, valvular disease, aortic regurgitation, HFpEF (heart failure with preserved ejection fraction), diastolic failure, systolic failure, supraventricular tachyarrhythmia, congestive heart failure, coronary artery disease, idiopathic cardiomyopathy, atrial fibrillation, or the like. Examples of mammals include humans, monkeys, cows, sheep, goats, horses, pigs, rabbits, dogs, cats, rats, mice, guinea pigs, and the like. In particular, humans having developed an intractable heart tissue fibrosis disease, or humans suspected to have developed the disease, are preferable.

The method for administering the pharmaceutical composition of the present invention is not particularly limited, as long as the method enables the active ingredient to reach the disease site. Examples of methods include oral administration, intravenous administration, drip/infusion administration, intracoronary administration, inhalation administration, intramuscular administration, subcutaneous administration, suppository, intraperitoneal/intrathoracic administration, transmucosal administration, transdermal administration, injectable preparations for internal organs, patch application administration, and the like. Oral administration is generally used. When the pharmaceutical composition is formed into a nanosphere preparation or a microsphere sustained-release preparation comprising a biodegradable polymer containing a pharmaceutical composition therein, examples of administration methods include intravenous administration, drip/infusion administration, intra-arterial administration, inhalation administration, intramuscular administration, subcutaneous administration, suppository, intraperitoneal/intrathoracic administration, transmucosal administration, transdermal administration, patches, injectable formulations for internal organs, organ patch application, and the like. Subcutaneous administration, intramuscular injection, organ patch application, intravenous injection, or inhalation administration is usually used.

### 9. Method for Administering the Pharmaceutical Composition of the Present Invention

The amount of the pharmaceutical agent or the like contained in the pharmaceutical composition of the present invention varies depending on the type of pharmaceutical agent, its dosage form, age, body weight, symptom, therapeutic effect, administration interval, or administration route. When the pharmaceutical composition is in the form of an oral preparation, the amount can be appropriately selected within the range lower than the maximum tolerated dose determined from the results of long-term toxicity studies and phase I clinical trials. The lower limit is not particularly limited; and any dose can be selected, as long as the desired effect can be provided.

For example, the clinical maximum dose is usually such that the daily amount of (B) camostat mesilate is 600 mg (10 mg/kg), and this amount is orally administered in 3 divided doses; the daily amount of (C) ozagrel hydrochloride hydrate is 400 mg (6.7 mg/kg), and this amount is orally administered in 2 divided doses; the daily amount of (D) cilostazol is 200 mg (3.3 mg/kg), and this amount is orally administered in 2 divided doses; the daily amount of (E) pirfenidone is 1,800 mg (30 mg/(kg)), and this amount is orally administered in 3 divided doses; and the daily amount of (G) nintedanib ethanesulfonate is 300 mg (5 mg/(kg)), and this amount is orally administered in 2 divided doses.

Further, the daily amount of (H) theophylline is 400 mg (6.7 mg/kg), and this amount is orally administered in 2 divided doses; (I) sildenafil is orally administered in an amount of 50 mg (0.83 mg/kg) once a day; and (J) lovastatin is orally administered in an amount of 20 mg/day (0.33 mg/kg).

The administration period is appropriately determined in consideration of safety, convenience, patient's burden, compliance, etc., based on the disease and therapeutic method therefor. The administration frequency is not limited, as long the effect can be expected and convenient administration intervals can be set; and the composition in the form of an oral preparation is preferably administered once a day, twice a day, or three times a day. The range from once-a-day to twice-a-day dosing intervals is more preferable.

Side effects in clinical trials are as follows.
(B) Camostat mesilate was observed to have no side effects, even when administered in a daily amount of 600 mg. However, 83 side effects (including laboratory test value abnormality) were observed in 69 (1.8%) cases out of 3,806 cases whose side effects were tallied in a survey until approval for the remission of acute symptoms in chronic pancreatitis, and in a post-marketing surveillance. The main side effects were 15 rash cases (0.4%), 9 itching cases (0.2%), 10 nausea cases (0.3%), 7 abdominal discomfort cases (0.2%), 6 sense of abdominal fullness cases (0.2%), etc. (at the end of reexamination).
(C) Ozagrel hydrochloride hydrate was confirmed to have no safety problems except for slightly prolonged bleeding time, even when administered in a daily amount of 400 mg. 194 side effects (including laboratory test value abnormality) were observed in 154 cases (2.0%) out of 7,694 cases whose side effects were tallied in a survey until approval, and in a post-marketing surveillance. The main side effects were 25 elevated AST (GOT) and/or ALT (GPT) cases (0.3%), 21 nausea cases (0.3%), 16 pruritus cases (0.2%), 12 rash cases (0.2%), 9 stomach and/or abdominal discomfort cases (0.1%), 9 bleeding tendency cases (0.1%), etc. (at the time of re-examination).
(D) Cilostazol was observed to have side effects, including laboratory test value abnormality, in 209 cases (6.3%) out of 3,335 cases that were subjected to safety analysis in a use-results survey on amelioration of various ischemic symptoms, such as ulcer, pain, coldness, etc., based on chronic arterial occlusion. The main side effects were headache/heavy-headedness (3.4%), palpitations (0.7%), dizziness (0.5%), diarrhea (0.3%), and nausea/vomiting (0.3%) (at the time of completion of re-examination for Pletaal tablets).
(E) Pirferidone was observed to have side effects in 233 cases (87.9%) out of 265 cases that were subjected to safety evaluation at the time of approval. The main side effects were 137 light-hypersensitivity cases (51.7%), 61 anorexia cases (23.0%), 37 gastric discomfort cases (14.0%), and 32 nausea cases (12.1%). Further, abnormal fluctuation in laboratory test values was observed in 120 cases (45.3%) out of 265 cases subjected to safety evaluation. The main side effect was 53 γ-GTP elevation cases (20.0%).
(G) Nintedanib ethanesulfonate was subjected to 2 trials in Phase III international joint study using 1061 subjects. In these 2 trials, this agent was administered to 638 subjects. These two trials were randomized, double-blind, placebo-controlled trials with 150 mg of the agent being administered twice a day for 52 weeks. The main side effects in the 2 trials were 342 diarrhea cases (53.6%), 122 nausea cases (19.1%), 67 liver enzyme cases (10.5%), and 65 abdominal pain cases (10.2%) (at the time of application for approval).
(H) Theophylline was observed to have side effects in 85 cases (9.05%) out of 939 cases that were subjected to safety analysis at the time of approval. The main side effects were 38 nausea cases (4.05%), 24 headache cases (2.56%), 14 abdominal pain cases (1.49%), 12 anorexia cases (1.28%), and 11 palpitations cases (1.17%).
(I) Sildenafil was observed to have side effects or laboratory test value abnormality in 65 cases (41.40%) out of 157 cases that were subjected to a domestic clinical trial at the time of approval. The main side effects or laboratory test value abnormality were 17 vasodilation (hot flashes, flushes) cases (10.83%), 17 headache cases (10.83%), 9 CK (CPK) elevation cases (5.73%), etc.
(J) Lovastatin was observed to have side effects, including laboratory test value abnormality, in 1950 cases (18.8%) out of 10380 cases that were subjected to side effect evaluation in clinical trials in Japan and overseas. The main side effects were 335 muscle pain cases (3.2%), 179 ALT (GPT) elevation cases (1.7%), and 179 CK (CPK) cases (1.6%) (at the time of approval).

On the other hand, microsphere sustained release preparations comprising the pharmaceutical agent are preferably administered subcutaneously or by intramuscular injection approximately once a week, once every two weeks, once every four weeks, or once every three months.

Nanosphere preparations comprising the pharmaceutical agent are preferably administered by intravenous injection or subcutaneous injection approximately once a day, once every three days, once a week, once every two weeks, or once every four weeks.

When the pharmaceutical agent of the present invention, or a combination drug of the pharmaceutical agent of the present invention and other medicine is to be administered, the pharmaceutical agent or the combination drug is used as an internal solid preparation, an internal liquid preparation, or like preparation for oral administration; an injectable preparation for parenteral administration; a subcutaneous and/or intramuscular injection preparation; a preparation for external use; a suppository; an inhalant; or the like.

A single dose in intermittent administration of these sustained-release preparations is not limited, as long as it is less than or equal to the total dose amount in oral administration. The dose is usually less than or equal to one-tenth of the total dose amount.

As the oral preparation of the present invention, a commercially available pharmaceutical preparation can be used as is. Examples of the internal solid preparation for oral administration include tablets, pills, capsules, powders, granules, and the like. Capsules include hard capsules and soft capsules. Such internal solid preparations are formulated in a usual manner by mixing one or more active materials *per se,* or in the form of various salts thereof with an excipient (e.g., lactose, mannitol, glucose, microcrystalline cellulose, starch, etc.), a binder (e.g., hydroxypropyl cellulose, polyvinyl pyrrolidone, magnesium aluminometasilicate, etc.), a disintegrant (e.g., calcium fibrinoglycolate, etc.), a lubricant (e.g., magnesium stearate, etc.), a stabilizer, and/or a solubilizer (e.g., glutamic acid, aspartic acid, etc.), and used. If necessary, the solid preparation may be coated with a coating agent (e.g., white sugar, gelatin, hydroxypropyl cellulose, hydroxypropyl methylcellulose phthalate, etc.), or may be coated with two or more layers. Further, the solid preparations include capsules made of an absorbable substance, such as gelatin.

Internal liquid preparations for oral administration include pharmaceutically acceptable solutions, suspensions, emulsions, syrups, elixirs, and the like. Such liquid preparations can be formed by dissolving, suspending, or emulsifying one or more active materials, or salts thereof, in a commonly used diluent (e.g., purified water, ethanol, or a mixture thereof). Such liquid preparations may further contain a wetting agent, a suspending agent, an emulsifier, a sweetener, a fragrance, a preservative, a buffer, and the like.

The long-acting preparation of the present invention is not limited in formulation form, as long as the active ingredient can be continuously supplied to cardiac tissue or the blood concentration of the active ingredient can be maintained. For example, a sustained-release preparation (e.g., a microcapsule preparation, a microsphere preparation, a nanosphere preparation, etc.) can be administered by subcutaneous injection, intramuscular injection, intravenous injection, or cardiac patch application.

The microcapsule preparation, the microsphere preparation, and the nanosphere preparation of the present invention are preferably microparticle pharmaceutical compositions comprising any effective component as an active ingredient, and a biodegradable polymer.

The drug sustained-release system of the present invention comprises a bioabsorbable polymer. More specifically, the drug sustained-release system of the present invention comprises a natural polymer or a synthetic polymer. The control mechanism for controlling the rate of sustained release from these polymers includes, for example, those of a degradation control type, a diffusion control type, and a membrane permeation control type.

Examples of natural polymers that can be used as the bioabsorbable polymer of the present invention include plant-produced polysaccharides (e.g., cellulose, starch, alginic acid, etc.), animal-produced polysaccharides and proteins (e.g., chitin, chitosan, collagen, gelatin, albumin, glucosaminoglycan, etc.), microbially produced polyesters and polysaccharides (e.g., poly-3-hydroxyalkanoate, hyaluronic acid, etc.), and the like.

Examples of the biodegradable polymer include polymers or copolymers of fatty acid esters, polyacrylic acid esters, polyhydroxybutyric acids, polyalkylene oxalates, polyorthoesters, polycarbonates, and polyamino acids. These polymers can be used singly, or in a combination of two or more. Examples of polymers or copolymers of fatty acid esters include polylactic acid, polyglycolic acid, polycitric acid, polymalic acid, polyethylene succinate, polybutylene succinate, poly-s-caprolactone, polybutylene terephthalate-adipate, and poly(lactic-co-glycolic acid). These polymers can be used singly, or as a mixture of two or more. Other examples include poly-α-cyanoacrylic acid ester, poly-β-hydroxybutyric acid, polytrimethylene oxate, polyorthoester, polyorthocarbonate, polyethylene carbonate, poly-γ-benzyl-L-glutamic acid, polyvinyl alcohol, polyester carbonate, polyacid anhydride, polycyanoacrylate, polyphosphazene, and poly-L-alanine. These compounds can be used singly, or in a combination of two or more. Polylactic acid, polyglycolic acid, and poly(lactic-co-glycolic acid) are preferable, and poly(lactic-co-glycolic acid) is more preferable.

These biodegradable polymers used in the present invention preferably have an average molecular weight of about 2,000 to about 800,000, and more preferably about 5,000 to about 200,000. For example, polylactic acid preferably has a weight average molecular weight of about 5,000 to about 100,000, and more preferably about 6,000 to about 50,000. Polylactic acid can be synthesized according to a known production method. The composition ratio of lactic acid and glycolic acid in the poly(lactic-co-glycolic acid) is preferably in the range of about 100/0 to about 50/50 (W/W), and particularly preferably in the range of about 90/10 to 50/50 (W/W). The poly(lactic-co-glycolic acid) preferably has a weight average molecular weight of about 5,000 to about 100,000, and more preferably about 10,000 to 80,000. The poly(lactic-co-glycolic acid) can be synthesized according to a known production method. A basic amino acid (such as alginic acid) or the like may be added in order to reduce the initial burst.

In the present specification, the weight average molecular weight refers to the molecular weight in terms of polystyrene as measured by gel permeation chromatography (GPC).

As long as the object of the present invention can be achieved, the biodegradable polymer to be used can be changed according to the potency of pharmacological activity of the active ingredient, and the desired drug release. For example, the amount of the biodegradable polymer to be used is about 0.2 to 10,000 times (mass ratio), preferably about 1 to 1000 times (mass ratio), and more preferably about 1 to 100 times (mass ratio), the amount of the biologically active substance.

Examples of methods for producing the microspheres, microcapsules, or nanocapsules of the present invention include in-water drying methods (e.g., o/w method, w/o method, w/o/w method, etc.), phase separation methods, spray-drying methods, granulation methods using supercritical fluid, and other methods equivalent to these methods.

Specific production methods of the in-water drying method (o/w method) and the spray-drying method are described below.

### (1) In-Water Drying Method (o/w Method)

In the in-water drying method, a solution of a biodegradable polymer in an organic solvent is first prepared. The organic solvent used in the production of microspheres, microcapsules, or nanocapsules of the present invention preferably has a boiling point of 120°C or less. Examples of the organic solvent include halogenated hydrocarbons (e.g., dichloromethane, chloroform, etc.), aliphatic esters (e.g., ethyl acetate etc.), ethers, aromatic hydrocarbons, ketones (e.g., acetone, etc.), and the like. Two or more of these organic solvents can be mixed at an appropriate ratio, and used. Examples of preferable organic solvents include dichloromethane and acetonitrile. The organic solvent is preferably dichloromethane. The concentration of the biodegradable polymer in the organic solvent solution varies depending on the molecular weight of the biodegradable polymer, the type of organic solvent, etc.; and is generally selected from the range of about 0.01 to about 80% (v/w), more preferably about 0.1 to 70% (v/w), and even more preferably from about 1 to 60% (v/w).

An active ingredient is added to the thus-obtained solution of a biodegradable polymer in an organic solvent, and dissolved. The amount of the active ingredient to be added varies depending on the type of drug, angiogenesis action, duration of the effect, etc. The concentration of the biodegradable polymer in the organic solvent solution is in the range of about 0.001% to about 90% (w/w), preferably about 0.01% to about 80% (w/w), and more preferably about 0.3 to 30% (w/w).

Subsequently, the organic solvent solution thus prepared is further added to an aqueous phase, and an o/w emulsion is formed by using a stirrer, an emulsifier, or the like. The aqueous phase volume at this time is usually selected from about 1 time to about 10,000 times, preferably about 2 times to about 5,000 times, and particularly preferably about 5 times to about 2,000 times, the volume of the oil phase. An emulsifier may be added to the outer aqueous phase. The emulsifier may be any emulsifying agent, as long as it can form a stable o/w emulsion. Examples of the emulsifier include anionic surfactants, nonionic surfactants, polyoxyethylene castor oil derivatives, polyvinyl pyrrolidone, polyvinyl alcohol, carboxymethyl cellulose, lecithin, gelatin, and the like. These emulsifiers can be used in combination, as appropriate. The concentration of the emulsifier in the outer aqueous phase is preferably about 0.001% to about 20% (w/w), more preferably about 0.01% to about 10% (w/w), and particularly preferably about 0.05% to about 5% (w/w).

For evaporation of the oil-phase solvent, a commonly used method is used. The method is carried out at ordinary pressure or gradually reduced pressure while stirring, using, for example, an agitator or a magnetic stirrer; or using a rotary evaporator or the like, while adjusting the degree of vacuum. After the thus-obtained microspheres are separated by centrifugation or filtration, substances adhered to the microsphere surface, such as free active ingredients and emulsifiers, are washed several times repeatedly with, for example, a surfactant solution, alcohol, or the like, and the resulting microspheres are dispersed again in distilled water or a dispersion medium containing an excipient (mannitol, sorbitol, lactose, etc.) and freeze-dried. The o/w method may be a method comprising dispersing an active ingredient in a solution of a biodegradable polymer in an organic solvent. That is, microspheres can be produced by the s/o/w method.

(2) When microspheres are produced by a spray-drying method, a solution of a biodegradable polymer and an active ingredient in an organic solvent, or an emulsion thereof, is sprayed into a drying chamber of a spray-dryer device (spray dryer) using a nozzle, and the organic solvent or water in microparticle droplets is evaporated in a very short time to prepare microspheres. Examples of the nozzle include nozzles of a double-fluid nozzle type, a pressure nozzle type, a rotary disc type, or the like. At the time of spraying, if desired, it is effective to spray an organic solvent or an aqueous solution of an aggregation-preventing agent (mannitol, lactose, gelatin, etc.) through another nozzle simultaneously with spraying of the o/w emulsion, in order to prevent aggregation of microspheres. The moisture and solvent in the thus-obtained microspheres are completely removed from the thus-obtained microspheres, if necessary, with heating.

Examples of film preparations include those obtained by dissolving a biodegradable polymer and an active ingredient in an organic solvent, followed by evaporation to dryness to form a film; those obtained by dissolving a biodegradable polymer and an active ingredient in an appropriate solvent, and then gelling the solution by adding a granulation agent (celluloses, polycarbonates, etc.); and the like.

The microspheres, microcapsules, and nanospheres of the present invention can be formulated into various dosage forms, for example, as is; or using a spherical, rod-, needle-, bolt-, thread-, pellet-, or film-shaped, or creamy pharmaceutical composition as a material substance.

Further, this pharmaceutical preparation can be administered as a parenteral preparation for topical administration (e.g., injectable preparations for intramuscular, subcutaneous, intradermal, intramyocardial, intraperitoneal, intrabronchial, intravascular, intrapulmonary, damaged vascular endothelium site, intracerebral, intramedullary, intradural, epidural, intra-articular, intraspinal, bone site, periodontal site, intraorgan, or organ surface administration; solid preparations such as implants, granules, and powders; liquid preparations such as suspensions; patches; film preparations; ointments; medical device-contained preparations comprising an active ingredient contained in medical devices (e.g., stents, bolts, surgical sutures, etc.), or active ingredient-containing coating agents with which medical devices are coated). Further, the pharmaceutical preparation can be directly administered to, for example, a myocardial ischemic site using a blood vessel catheter or the like.

For example, when microspheres are to be formed into an injectable formulation, microspheres are suspended with, for example, a dispersing agent, a preservative, an tonicity agent, a buffer, a pH adjusting agent, or the like, to form an aqueous suspension, thus obtaining a practical injectable preparation. Alternatively, microspheres can be dispersed with plant oil or a mixture of plant oil with a phospholipid, such as lecithin; or with a middle-chain fatty acid triglyceride (e.g., Miglyol 812) to form an oily suspension, thus obtaining a practical injectable preparation.

The particle size of the microspheres, for example, for use as a suspension injection, is not limited, as long as it is within a range such that the degree of dispersion and through-needle properties are satisfactory. For example, the average particle size is within the range of about 0.1 to about 300 µm, preferably about 1 to 150 µm, and more preferably about 2 to 100 µm. The pharmaceutical composition of the present invention is preferably a suspension as described above. The pharmaceutical composition of the present invention is preferably in the form of microparticles. This is because administration of the pharmaceutical composition through an injection needle used for a usual subcutaneous or intramuscular injection does not cause patients excessive pain. The pharmaceutical composition of the present invention is particularly preferable as an injectable preparation. When the microspheres are formed into sterile preparations, examples of usable methods include, but are not limited to, a method in which all of the production steps are performed under aseptic conditions, a method in which microspheres are sterilized with gamma rays, and a method in which an antiseptic is added.

The pharmaceutical composition of the present invention has a sustained-release property in the action of the active ingredient. The sustained release period varies depending on the kind, blending amount, etc., of the biodegradable polymer, and is usually 1 week to 3 months. Therefore, the pharmaceutical composition of the present invention can be used as a sustained-release preparation that gradually releases various types of compounds at (ischemic) organ damage sites, and that maintains a high concentration of the active ingredient at the damage sites.

The dosage amount of the pharmaceutical composition of the present invention varies depending on the kind and content of active ingredient, dosage form, duration of drug release, subject animal to which the composition is administered, etc.; however, it may be any amount, as long as an effective amount of the active ingredient is released. For example, when the composition is used as microspheres at an ischemic site, it can be administered at a dose, in terms of the active ingredient, of about 0.001 mg to 500 mg, preferably about 0.01 mg to 100 mg, per adult (body weight: 50 kg), once a day to once every 3 months.

In the present invention, it is also preferable to administer two or more kinds of drugs selected from these compounds; antihypertensive agents such as currently used β blockers, ARB, and ACE inhibitors; prostaglandin IP receptor agonists, prostaglandin EP2 receptor agonists, and prostaglandin EP4 receptor agonists in combination, according to the purpose. These drugs are commercially available, or can be easily produced according to known methods.

The administration route of the present invention is mainly oral administration, because it is a long-term administration. However, microsphere preparations comprising active substances are mainly administered by parenteral administration, such as intermittent subcutaneous administration or intermittent intramuscular injection. Nanosphere preparations are mainly administered by intermittent intravenous injection, intermittent subcutaneous injection, intermittent intramuscular injection, or the like.

Since microsphere preparations and nanosphere preparations, which are sustained-release preparations, are parenteral administrations, these preparations are suitable for application to compounds having low absorptivity and low bioavailability (BA) in oral administration. When compounds requiring intravenous drip infusion are administered, the compound exhibits long-term intravenous infusion-like blood kinetics by intermittent subcutaneous administration, intermittent intramuscular injection, or the like, thus increasing efficacy due to improved convenience in administration, side-effect avoidance, and sustained effects.

When nanospheres are used, improvement in oral absorption can also be expected.

For example, since most of camostat mesilate is deactivated by gastrointestinal esterase, camostat mesilate itself is modified to a prodrug; however, the absorption rate of the active substance is still low. Accordingly, intermittent subcutaneous administration or intramuscular administration of a poly(lactic-co-glycolic acid) microsphere (PLGA·MS) preparation significantly improves efficacy for sustained maintenance of active substance concentration in the blood.

Similarly, sivelestat sodium hydrate as a drip injection exhibits long-term intravenous infusion-like blood dynamics in intermittent subcutaneous administration in the form of a PLGA·MS preparation, thus improving convenience; and also exhibits efficacy for chronic diseases, such as chronic obstructive pulmonary disease (COPD). Further, when nanospheres are used, improved oral absorption and local accumulation at a disease site can also be expected.

The present invention, as a new method for treating patients with severe heart disease, can be expected to improve life prognosis, and significantly enhance QoL. Further, a cardiovascular and myocardial regeneration therapy, which enables a combination of these drugs to delay the progression of heart failure, and thereby avoid and delay heart transplant and/or LVAD implantation, is a ground-breaking new therapy for heart failure. Furthermore, the present invention inhibits the aggravation of a disease by early therapeutic intervention, and also enables a definitive treatment at a lower cost than heart transplant or LVAD; it is also expected to contribute to the medical economy.

Therapeutic research on intractable diseases and specified diseases, such as dilated cardiomyopathy, has not gained attention. Because of rare diseases, research on these diseases has been left behind. The present invention contributes to solving this unsolved problem.

Based on the development of a minimally invasive, versatile, economical cardiovascular/myocardial regeneration therapeutic agent as a replacement for a left ventricular assist device, heart transplant, and cell therapy, which are methods for treating dilated cardiomyopathy and intractable heart disease, i.e., rare diseases, the present invention enables heart transplant or LVAD implantation to be delayed or avoided through early therapeutic intervention by oral administration. The present invention is also useful for promoting LVAD removal after LVAD implantation, or as a maintenance therapy after bypass surgery in ischemic cardiomyopathy.

A protease inhibitor, a phosphodiesterase (PDE) inhibitor, a tyrosine kinase inhibitor, a thromboxane (TX) A₂ synthase inhibitor, an HMG-CoA reductase inhibitor, and an antifibrotic agent, which are compounds of the present invention, were evaluated by administration at doses determined based on the maximum no-observed-adverse-effect level (NOAEL) in a rat long-term oral administration toxicity study; as well as clinical doses, using heart disease model animals, such as rat coronary artery complete ischemia models and spontaneous dilated cardiomyopathy hamster models. The results show that all of these compounds exhibited significantly higher efficacy than vehicle-control groups. These PLGA·MS preparations exhibited efficacy in intermittent subcutaneous administration once every 4 weeks in an amount that is not more than one-tenth of the total dose amount of repeated oral administration. These compounds can be administered in combination with a β blocker, ARB, ACE inhibitor, or the like currently clinically used as an antihypertensive agent. These compounds are all commercially available as oral pharmaceutical preparations, and are confirmed to be safe for humans.

### Examples

The present invention is described in more detail below with reference to Examples, but is not limited to these Examples.

The pharmaceutical products and compounds shown in the Examples are as described below. In the Examples, these products and compounds may be described by their abbreviations (generic names that do not include salt) or compound designations (A to O, B·MS, C·MS, and I·MS).

These are commercially available from the following companies, and can generally be purchased. Table 1 shows the name, mechanism of action, indication, and the name of the commercial source of each test substance.

### Test substances

(1) Prostaglandin IP receptor agonists:
   i) (A) ONO-1301 (CAS: 176391-41-6) (Ono Pharmaceutical Co., Ltd./Sigma-Aldrich)
   ii) (F) Beraprost sodium (CAS: 88475-69-8) (Astellas Pharma Inc./Cayman)
(2) Protease inhibitor: (B) Camostat mesilate (CAS: 59721-29-8) (Ono Pharmaceutical Co., Ltd./Wako Pure Chemical Industries, Ltd.)
(3) Thromboxane A2 synthase inhibitor: (C) Ozagrel hydrochloride hydrate (CAS: 78712-43-3) (Ono Pharmaceutical Co., Ltd./Tokyo Chemical Industry Co., Ltd.)
(4) Phosphodiesterase (PDE) inhibitors:
   i) PDE III inhibitor: (D) Cilostazol (CAS: 73963-72-1) (Otsuka Pharmaceutical Co., Ltd./Tokyo Chemical Industry Co., Ltd.)
   ii) PDEV inhibitor: (I) Sildenafil citrate (CAS: 171599-83-0) (Pfizer/SIGMA)
   iii) Nonselective PDE inhibitor: (H) Theophylline (CAS: 58-55-9) (Otsuka Pharmaceutical Co., Ltd./Nacalai Tesque)
(5) Antifibrotic agent: (E) Pirfenidone (CAS: 53179-13-8) (Shionogi & Co., Ltd./Tokyo Chemical Industry Co., Ltd.)
(6) Tyrosine kinase inhibitor: (G) Nintedanib ethanesulfonate (CAS: 656247-18-6 (Boehringer Ingelheim Japan, Inc./LC Laboratories)
(7) HMG-CoA reductase inhibitor: (J) Lovastatin calcium (CAS: 147098-20-2) (AstraZeneca/Tokyo Chemical Industry Co., Ltd.)
(8) (K) Candesartan cilexetil (ARB) (CAS: 145040-37-5) (Takeda Pharmaceutical Company Limited/Tokyo Chemical Industry Co., Ltd.)
(9) (O) Carvedilol (CAS: 72956-09-3) (Pfizer/Tokyo Chemical Industry Co., Ltd.)

**Table 1**

| Generic name | Mechanism of action | Target disease | |
|---|---|---|---|
| (B) Camostat | Protease inhibitor | Remission of acute symptoms in chronic pancreatitis, and postoperative reflux esophagitis | Ono Pharmaceutical Co., Ltd. etc. |
| (E) Pirfenidone | TGF-β reduction | Idiopathic pulmonary fibrosis | Shionogi & Co., Ltd. |
| (G) Nintedanib | Tyrosine kinase inhibitor | Idiopathic pulmonary fibrosis | Boehringer Ingelheim |
| (D) Cilostazol | PDE III inhibitor | Preparation for ameliorating ischemic symptoms, such as ulcer, pain, and cold sensation based on chronic arterial occlusion; and recurrence inhibitor after the onset of cerebral infarction (except for cardiogenic embolism) | Otsuka Pharmaceutical Co., Ltd. etc. |
| (C) Ozagrel | TXA2 synthase inhibitor | Bronchial asthma | Kissei Pharmaceutical Co., Ltd./Ono Pharmaceutical Co., Ltd., etc. |
| (I) Sildenafil | PDEV inhibitor | Erectile dysfunction medication | Pfizer |
| (H) Theophylline | Nonselective PDE inhibitor | Bronchial asthma, asthmatic (asthma-like) bronchitis, chronic bronchitis, and emphysema | Otsuka Pharmaceutical Co., Ltd. etc. |
| (J) Lovastatin | HMG-CoA reductase inhibitor | Hypercholesterolemia and familial hypercholesterolemia drugs | AstraZeneca etc. |

### Dose-setting basis

The dose-setting basis for the doses of the test substances used in pharmacological tests is as described below. Supporting data therefor were taken from the background materials of the present inventors, and interview forms for the test substances.

### (A) ONO-1301

The minimal effective dose in the case of using spontaneous dilated cardiomyopathy (J2N-k) hamsters was 0.3 to 1 mg/kg. The no-observed-adverse-effect level in repeated oral administration in rats and dogs was 3 mg/kg. The dose was thus set to 3 mg/kg twice per day, as a dose in which the effect can be reliably confirmed.

### (B) Camostat mesilate

Mice with ethionine-induced pancreatitis were prepared by administering ethionine to mice fed a choline-deficient diet. Orally administration at 20 to 300 mg/kg twice daily to the prepared mice suppressed an increase in protease activity in the pancreas (300 mg/kg), and decreased the mortality (20 to 300 mg/kg).

Since suppression of body weight gain was observed at 550 mg/kg or more in repeated oral administration in rats for 6 months, the maximum no-observed-adverse-effect level was 235 to 550 mg/kg. The dose was thus set to 150 mg/kg twice per day.

### (C) Ozagrel hydrochloride hydrate

Antigen-induced airway constriction in sensitized rats and sensitized guinea pigs was suppressed by oral or intraduodenal administration at 100 mg/kg and 300 mg/kg.

In repeated oral administration in rats for 3 months, increases in Na, K, and white blood cells in the urine, and a decrease in serum Ca were observed at 500 mg/kg or more; and the no-observed-effect level was 150 mg/kg. The dose was thus set to 50 mg/kg twice per day.

### (D) Cilostazol

In a mouse model of pulmonary embolism induced by ADP or collagen injection, pulmonary embolus death was significantly inhibited by pre-administration (oral) of cilostazol at 30 mg/kg to the ADP-induced model, or at 10 mg/kg to the collagen-induced model.

In repeated oral administration in rats for 13 weeks, increased liver weight was observed at a high dose, and the no-observed-adverse-effect level was 30 mg/kg. The dose was thus set to 30 mg/kg twice per day.

### (E) Pirfenidone

In a BLM-induced mouse pulmonary fibrosis model, pirfenidone suppresses, in a dose-dependent manner, an increase in the amount of hydroxyproline associated with BLM administration, and the minimal effective dose for antifibrosis was 30 mg/kg/day.

In repeated oral administration in rats for 1 month, an increase in liver drug-metabolizing enzyme activity was observed at 100 mg/kg or more, and the no-observed-adverse-effect level was 100 mg/kg. The dose was thus set to 50 mg/kg twice per day.

### (F) Beraprost sodium

In a lauric acid-induced rat hind limb circulatory disorder model, the disorder of the hind limbs was significantly ameliorated by oral administration at 300 µg/kg/day for 7 consecutive days.

In a 12-month repeated oral administration toxicity study in rats, flushing etc. in the limbs, pinna, and tip of the nose were observed at 0.1 mg/kg/day or more; and the no-observed-effect level was 0.01 mg/kg/day. The maximum dose was thus set to 0.1 mg/kg twice per day.

### (G) Nintedanib ethanesulfonate

In bleomycin-induced pulmonary fibrosis model mice, suppression action on pulmonary fibrosis and pulmonary inflammation provided by prophylactic administration of nintedanib (administration from Day 0 to Day 14 after administration of bleomycin), and therapeutic administration of nintedanib (administration from Day 7 to Day 21 after administration of bleomycin) was investigated. Nintedanib was administered once daily by oral gavage at a dose of 30 mg/kg, and at a dose of 60 mg/kg. The results showed that in the prophylactic administration, a slightly higher inhibitory effect on pulmonary fibrosis was observed in the 60 mg/kg administration than in the 30 mg/kg administration. In the therapeutic administration, an apparent effect on both inflammation and fibrosis was observed in only the 60 mg/kg administration.

In a 6-month repeated administration toxicity study in rats, decreased red blood cell count, decreased PCV, decreased hemoglobin, decreased organ weights (the thymus and adrenal glands), etc., were observed at 20 mg/kg/day; and the no-observed-adverse-effect level was 5 mg/kg. The dose was thus set to 5 mg/kg twice per day.

### (H) Theophylline

When theophylline was orally administered to Fischer 344 rats for 13 weeks, an increase in periarteritis of arteries near the mesenteric lymph nodes, and an increase in mean corpuscular hemoglobin (MCH) were observed from 37.5 mg/kg. The maximum dose was thus set to 20 mg/kg twice per day.

### (I) Sildenafil citrate

Sildenafil was orally administered to SD rats for 6 months. As a result, increased liver weight, centrilobular hypertrophy of hepatocytes, and hypertrophy of thyroid follicular epithelium were observed in the 60 mg/kg group. The no-observed-adverse-effect level was 60 mg/kg/day. The dose was thus set to 30 mg/kg twice per day.

### (J) Lovastatin calcium

In 1-month, 3-month, and 6-month oral repeated administration studies in rats, the no-observed-adverse-effect levels were 15 mg/kg, 10 mg/kg, and 2 mg/kg, respectively. The dose was thus set to 5 mg/kg twice per day.

### (K) Candesartan cilexetil

Oral administration to spontaneously hypertensive rats (SHR) at 0.1, 1, and 10 mg/kg for 2 weeks reduced blood pressure, and increased the plasma renin concentration in a dose-dependent manner. The no-observed-adverse-effect level was 10 mg/kg in a 6-month toxicity study in rats, and 20 mg/kg in a 6-month toxicity study in dogs. The no-observed-adverse-effect level was thus set to 3 mg/kg once per day.

### (O) Carvedilol

Carvedilol, which acts to block both α1 and β receptors, was developed as a therapeutic agent for high blood pressure and angina pectoris. After further development, action on chronic heart failure and tachycardiac atrial fibrillation was confirmed. The drug has a β-blocking action as its main action, and the antihypertensive effect is mainly based on this action. However, blocking β receptors may increase the α1 receptor action of an endogenous catecholamine, causing vasoconstriction. To suppress this, the drug also has an α1 receptor blocking action. The dose was the clinical dose.

The following biodegradable polymer-encapsulated microsphere preparations can be produced as Preparations 1 to 3 in the Production Example of a PLGA·MS long-acting preparation described later.

The dose-setting basis is as described below.

### (B·MS) Camostat MS (Preparation 2)

The LD₅₀ for subcutaneous administration of camostat in rats was 1329 mg/kg; thus, intermittent subcutaneous administration at about 1/10 thereof, i.e., 100 mg/kg, once every four weeks was set. Moreover, the clinical dose of active substance camostat was 200 mg three times per day, i.e., 600 mg/day in total; thus, intermittent subcutaneous administration at 1/28 thereof, i.e., 10 mg/kg, once every four weeks was set.

### (C·MS) Ozagrel MS (Preparation 1)

The LD₅₀ for subcutaneous administration of ozagrel in rats was 2049 mg/kg; thus, intermittent subcutaneous administration at 1/40 thereof, i.e., 50 mg/kg, once every four weeks was set.

### (I·MS) Sildenafil MS (Preparation 3)

The lethal dose was 10 mg/kg or more in intravenous injection of sildenafil in rats, and 1000 mg/kg or more in oral administration; thus, intermittent subcutaneous administration at 1/30 thereof, i.e., 30 mg/kg, once every four weeks was set.

**Table 2**

| Candidate drugs that exert no antihypertensive action (combined use with O: β blocker) | | | | | |
|---|---|---|---|---|---|
| Designation | Generic name (oral preparation) | Maximum clinical dose mg/person/day (P.O.) | Clinical dose mg/kg/day | Maximum no-observed-adverse-effect level* mg/kg twice per day (screening 1) | Minimal effective dose** mg/kg twice per day (screening 2) |
| B | Camostat | 600 | 10 mg/kg | 150 mg/kg | 5 mg/kg |
| | | 200 mg three times | | | |
| G | Nintedanib | 300 | 5 mg/kg | 5 mg/kg | 2.5 mg/kg |
| | | 150 mg twice | | | |
| E | Pirfenidone | 1800 | 30 mg/kg | 50 mg/kg | 15 mg/kg |
| | | 600 mg three times | | | |
| C | Ozagrel | 400 | 6.7 mg/kg | 50, 10 mg/kg | 3.3 mg/kg |
| | | 200 mg twice | | | |
| D | Cilostazol | 200 | 3.3 mg/kg | 30 mg/kg | 1.7 mg/kg |
| | | 100 mg twice | | | |
| I | Sildenafil | 50 | 0.83 mg/kg | 30 mg/kg | 0.4 mg/kg |
| | | 50 mg once | | | |
| B·MS | Camostat MS | - | - | 100 mg/kg/4w (s.c.) | MS: 10 mg/kg/4w (s.c.) |
| O | Carvedilol | 20 | 0.33 mg/kg | - | 0.17 mg/kg |
| | | 20 mg once | | | |

Results of pharmacological tests are described below in more detail.

### 1. Investigation of effects in spontaneous dilated cardiomyopathy (J2N-k) hamster model

The doses of pharmaceutical products were determined based on the no-observed-adverse-effect levels in a long-term toxicity study using rats. δ-Glycan-deficient spontaneous dilated cardiomyopathy (J2N-k) male hamsters (Japan SLC, Inc.) were delivered at 18 weeks of age. After a 2-week
quarantine/acclimation, echocardiography was performed. Animals whose EF value was 25% or less, or 55% or more were excluded; and the other animals were grouped by stratified random assignment based on the EF values and the body weights so that each administration group was equalized.

Each of the pharmaceutical products (A to K and O) was individually administered twice daily (at an interval of 8 hours or more) by oral gavage, and echocardiography was performed after 4 and 8 weeks (before dissection). Differences from the value before the start of administration (at the time of grouping) (amount of change: Δ) in each group were examined, and the cardiac function (EF value, FS%, etc.) was compared with that in the vehicle group (Group 1). Moreover, the heart was removed 8 weeks after the start of administration, and electron microscopy and RNA/protein measurement were performed. Specimens for immunopathology were also collected and evaluated.

Changes in body weight and changes in cardiac function in three tests are described in detail below.

### 1) Test 1

Effects of the no-observed-adverse-effect levels of A to E were investigated.

### 2) Table 3 shows the constitution of the groups.

**Table 3**

| Group | Test substance | Dose (twice per day)* | Number of animals |
|---|---|---|---|
| 1 | Control (vehicle) | - | 8 to 10 |
| 2 | A (ONO-1301) | 3 mg/kg | 8 |
| 3 | B (Camostat) | 150 mg/kg | 8 |
| 4 | C (Ozagrel) | 50 mg/kg | 8 |
| 5 | D (Cilostazol) | 30 mg/kg | 8 |
| 6 | E (Pirfenidone) | 50 mg/kg | 8 |

| | | | |
|---|---|---|---|
| *: 8-week repeated oral gavage administration was performed at the doses described above twice per day, with an interval of 8 hours or more between the first administration and the second administration. The dose volume was 5 mL/kg (per dose). | | | |

Each pharmaceutical product was individually suspended in 0.5% CMC-Na (sodium carboxymethylcellulose; Wako Pure Chemical Industries, Ltd.) before use, and administered. Test substance B was suspended in 0.5% MC (methylcellulose 400; Wako Pure Chemical Industries, Ltd.) before use, and administered. To Group 1 (control), 0.5% CNC-Na (vehicle) was administered.

### Results

### (1) Changes in body weight (Table 4)

Each of (A) the ONO-1301 administration group, (B) the camostat mesilate administration group, (C) the ozagrel hydrochloride hydrate administration group, (D) the cilostazol administration group, and (E) the pirfenidone administration group showed a body weight change similar to that of the control group until 8 weeks after the start of administration of each test substance; and it was confirmed that there was no development of toxicity.

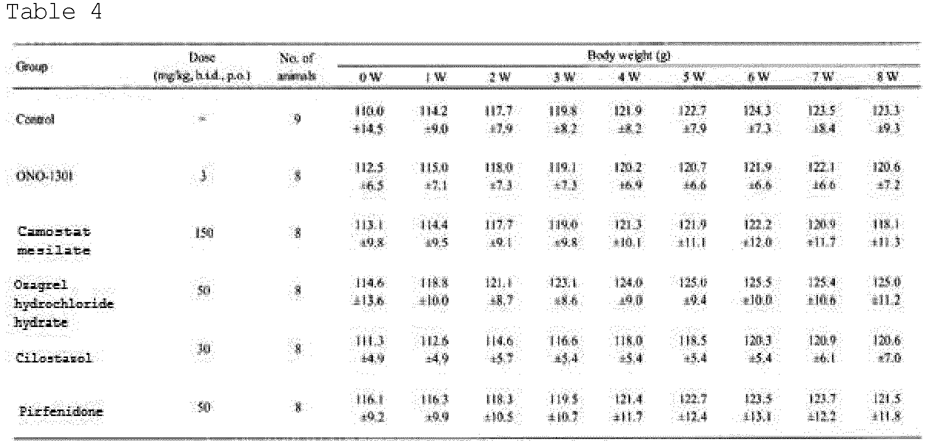

Each value represents the mean±S.D.

No significant difference was observed compared with the control (Student's t-test or Aspin-Welch's test).

### (2) Cardiac function test (EF value) (Table 5)

At the time of grouping (0W): No significant difference was observed in any of the administration groups of A to E, compared with the control group.

4W value: Both the actual measured value and the amount of change were significantly higher in each of the administration groups of A to E, than in the control group (P < 0.01 vs Cont group).

8W value: Both the actual measured value and the amount of change were significantly higher in each of the administration groups of A to E, than in the control group (P < 0.01 vs Cont group).

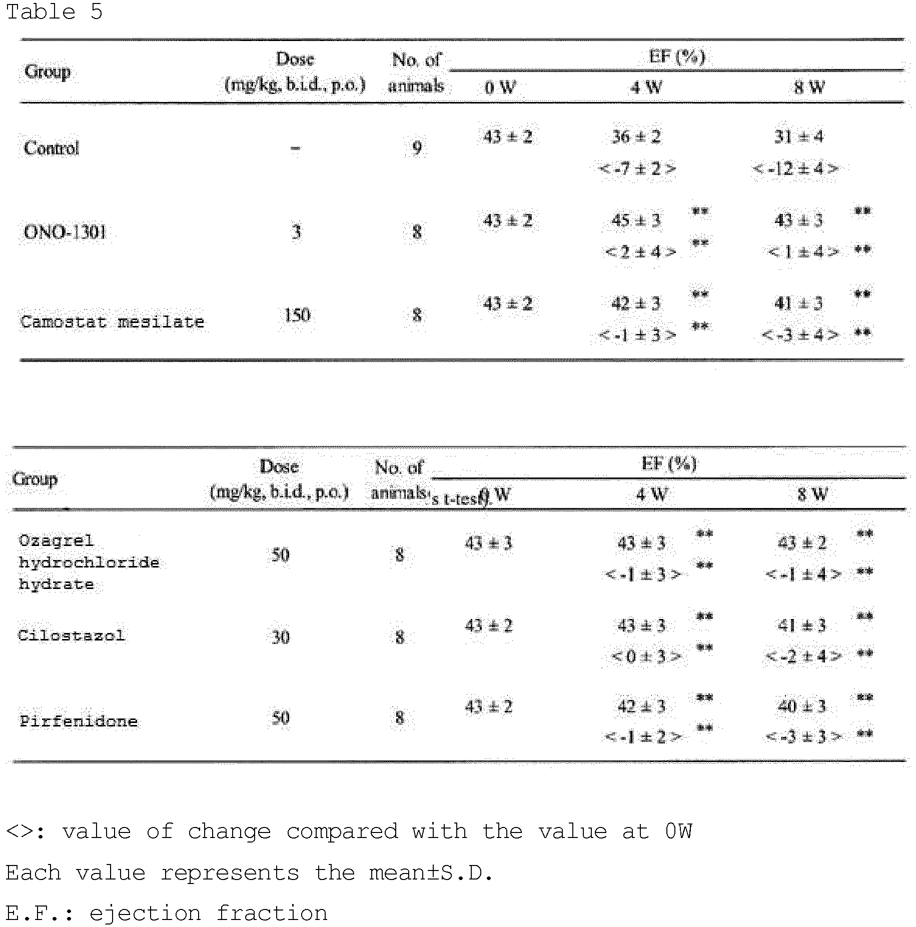

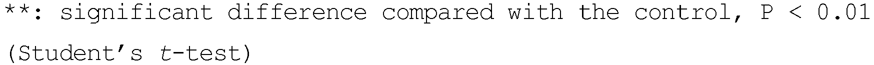

### (3) Cardiac function test (FS%) (Table 6)

At the time of grouping (0W): No significant difference was observed in any of the administration groups of A to E, compared with the control group.

4W value: Both the actual measured value and the amount of change were significantly higher in each of the administration groups of A to E, than in the control group (P < 0.01 vs Cont group).

8W value: Both the actual measured value and the amount of change were significantly higher in each of the administration groups of A to E, than in the control group (P < 0.01 vs Cont group).

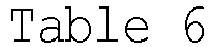

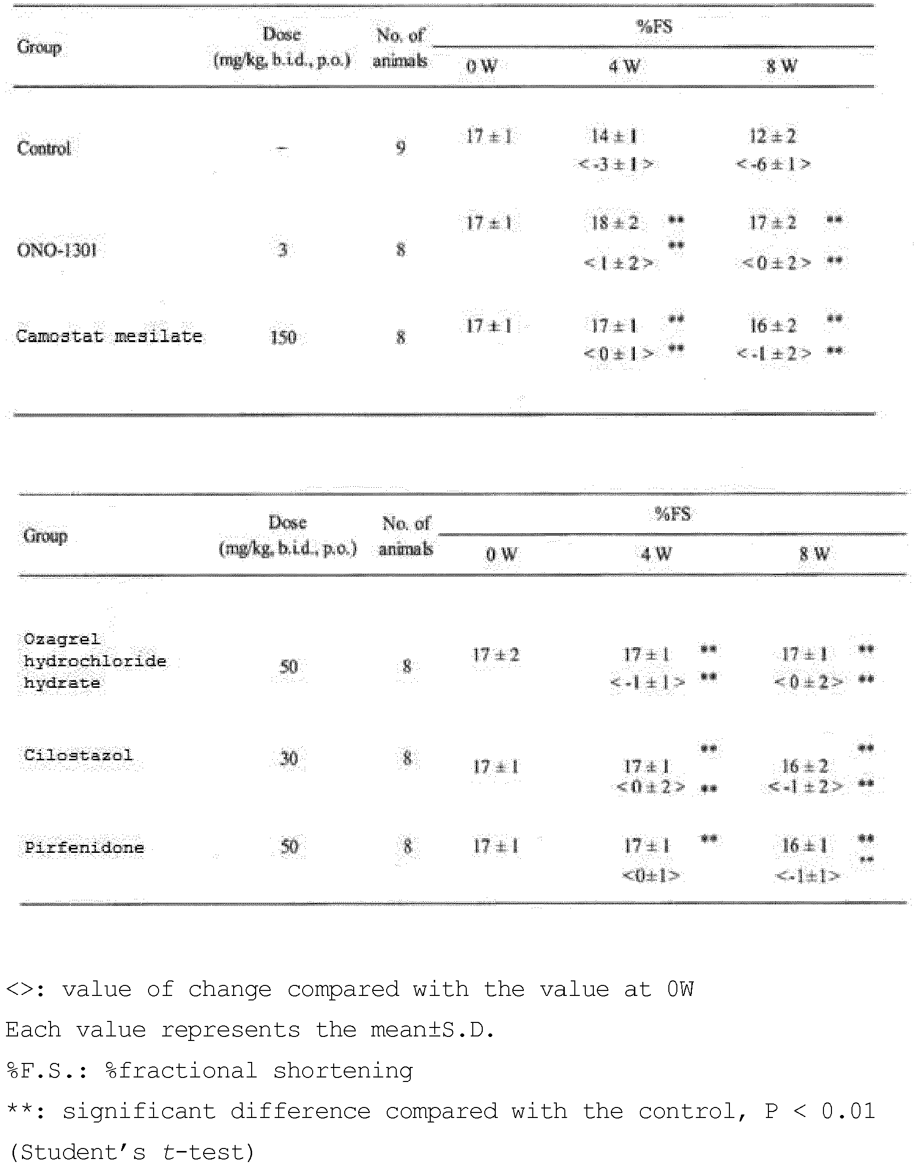

### (4) Lumen wall thickness (systolic left ventricular internal dimension (LVIDs)) (Table 7)

The groups of (B) camostat, (C) ozagrel, and (E) pirfenidone showed significantly lower actual measured values of LVIDs, compared with the control group.

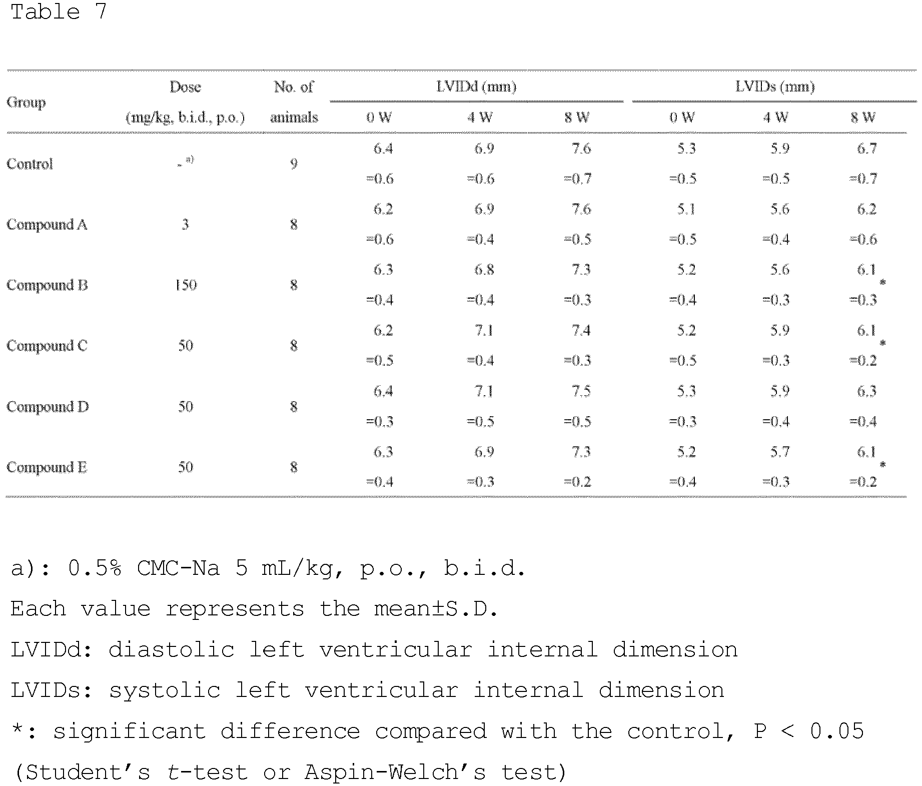

The results of individually orally administering each of the pharmaceutical products (A to E) repeatedly for 8 weeks twice daily, after the onset of pathological condition, i.e., from the age of 20 weeks until the age of 28 weeks, in the spontaneous dilated cardiomyopathy (J2N-k) hamster model revealed that all of them showed an action of significantly improving cardiac function, or suppressing deterioration of cardiac function in terms of EF and FS% of cardiac function. No significant impact on the body weight was observed in any of the groups.
(A) ONO-1301, used as a test substance, was set as a positive control in this model. (B) camostat mesilate, (C) ozagrel hydrochloride hydrate, (D) cilostazol, and (E) pirfenidone have already been used as pharmaceutical products for diseases. The results of administration of these substances based on the no-observed-adverse-effect levels determined from the toxicity study therefor confirmed that these pharmaceutical products are also useful for dilated cardiomyopathy.

### 1) Test 2

Effects of the no-observed-adverse-effect levels of F to J were investigated.

### 2) Table 8 shows the constitution of the groups.

**Table 8**

| Group | Test substance | Dose (twice per day) * | Number of animals |
|---|---|---|---|
| 1 | Control (vehicle) | (vehicle × 2) | 8 to 10 |
| 2 | F (beraprost) | 0.1 mg/kg | 8 |
| 3 | G (nintedanib) | 5 mg/kg | 8 |
| 4 | H (theophylline) | 20 mg/kg | 8 |
| 5 | I (sildenafil) | 30 mg/kg | 8 |
| 6 | J (lovastatin) | 5 mg/kg | 8 |

| | | | |
|---|---|---|---|
| *: 8-week repeated oral gavage administration was performed at the doses described above twice per day, with an interval of 8 hours or more between the first administration and the second administration. The dose volume was 5 mL/kg (per dose). | | | |

Each pharmaceutical product was individually suspended in 0.5% CMC-Na (sodium carboxymethylcellulose; Wako Pure Chemical Industries, Ltd.) before use, and administered. Test substance G was suspended in distilled water before use, and administered.

### Results

### (1) Changes in body weight (Table 9)

Each of (F) the beraprost administration group, (G) the nintedanib administration group, (H) the theophylline administration group, and (I) the sildenafil administration group showed a body weight change similar to that of the control group until 8 weeks after the start of administration of each test substance; and it was confirmed that no side effects occurred. In contrast, (J) the lovastatin administration group showed a body weight lower than that in the control group from 5 weeks after the start of administration of the test substance, and a body weight significantly lower by about 10% than that in the control group 8 weeks after the start of administration.

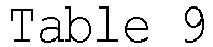

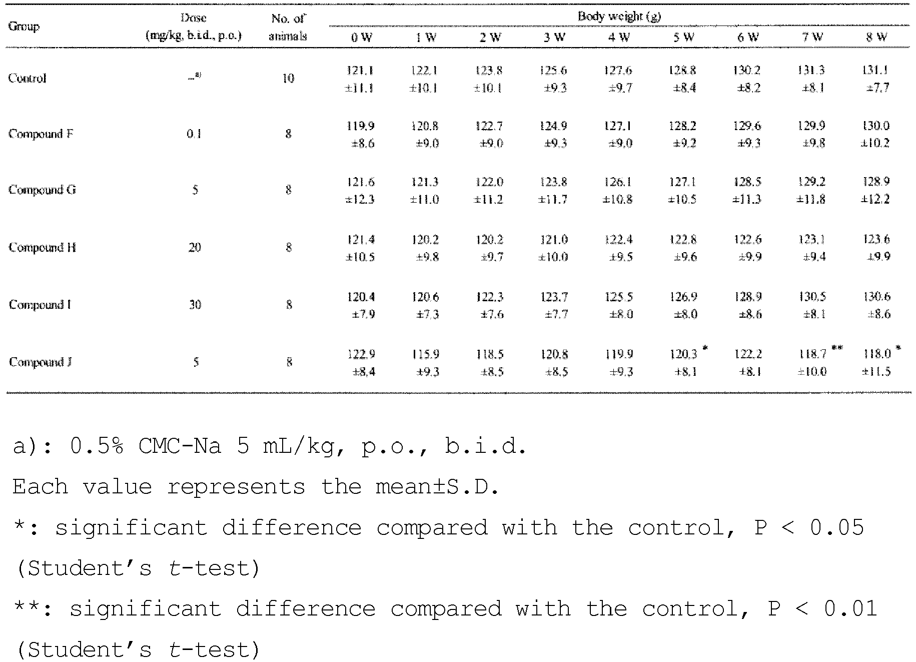

### (2) Cardiac function test (EF value) (Table 10)

At the time of grouping (0W): No significant difference was observed in any of the administration groups of F to J compared with the control group.

4W value: The actual measured value was significantly higher in each of (F) the beraprost administration group, (G) the nintedanib administration group, and (I) the sildenafil administration group, i.e., the administration groups other than (H) the theophylline administration group and (J) the lovastatin administration group, than in the control group. The amount of change was significantly higher in all of the administration groups other than (H) the theophylline administration group, than in the control group.

8W value: Both the actual measured value and the amount of change were significantly higher in each of the administration groups of F to J, than in the control group.

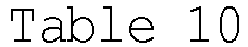

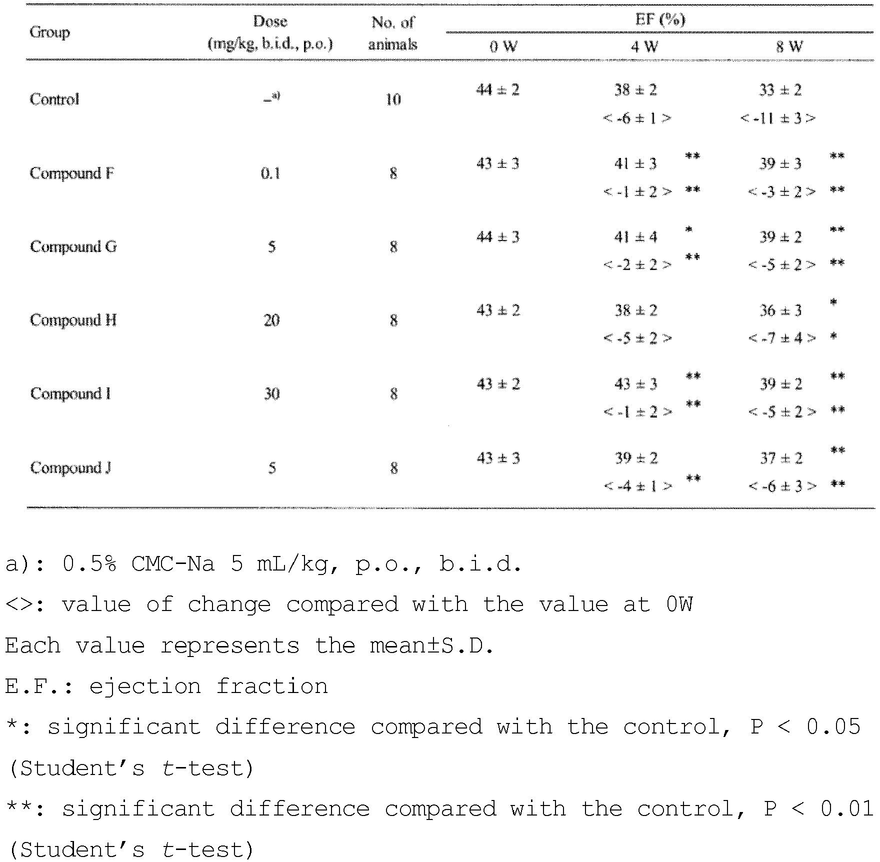

### (3) Cardiac function test (FS%) (Table 11)

At the time of grouping (0W): No significant difference was observed in any of the administration groups of F to J compared with the control group.

4W value: The actual measured value and the amount of change were significantly higher, than in the control group, in each of (F) the beraprost administration group, (G) the nintedanib administration group, and (I) the sildenafil administration group; however, the actual measured value and the amount of change were not significantly higher in (H) the theophylline administration group than in the control group, and the actual measured value was not significantly higher in (J) the lovastatin administration group than in the control group. In (J) the lovastatin administration group, only the amount of change was significantly higher than in the control group.

8W value: The actual measured value and the amount of change were significantly higher in each of the administration groups of F to J, than in the control group.

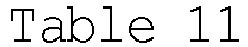

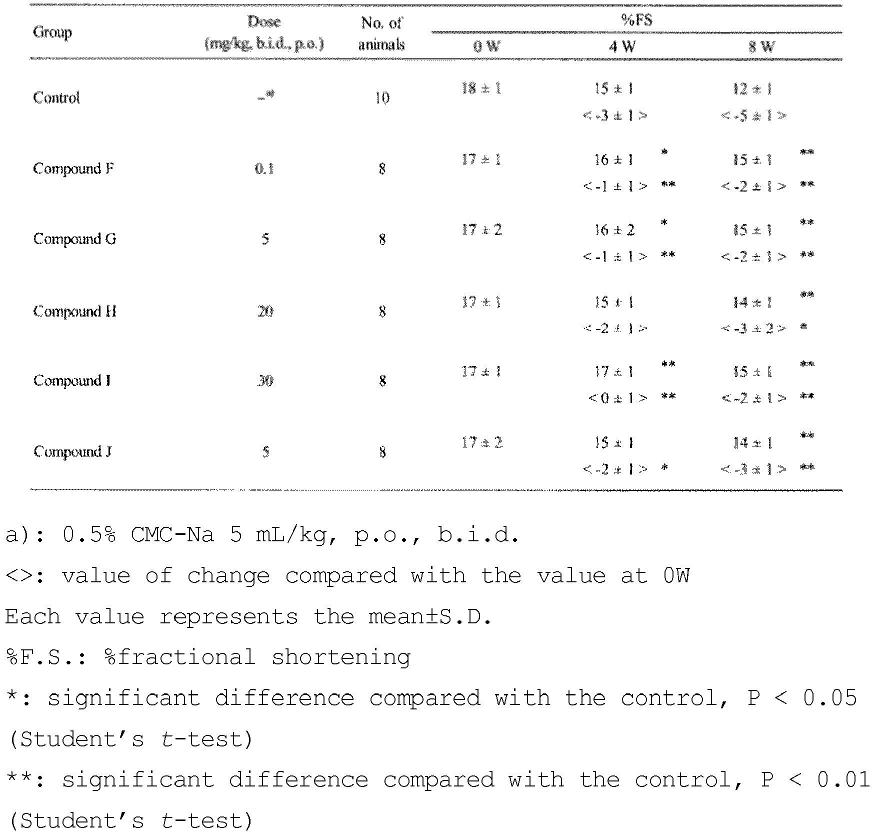

### (4) Lumen wall thickness (systolic left ventricular internal dimension (LVIDs)) (Table 12)

The groups of (G) nintedanib, (I) sildenafil, and (J) lovastatin showed significantly lower actual measured values of LVIDs, compared with the control group.

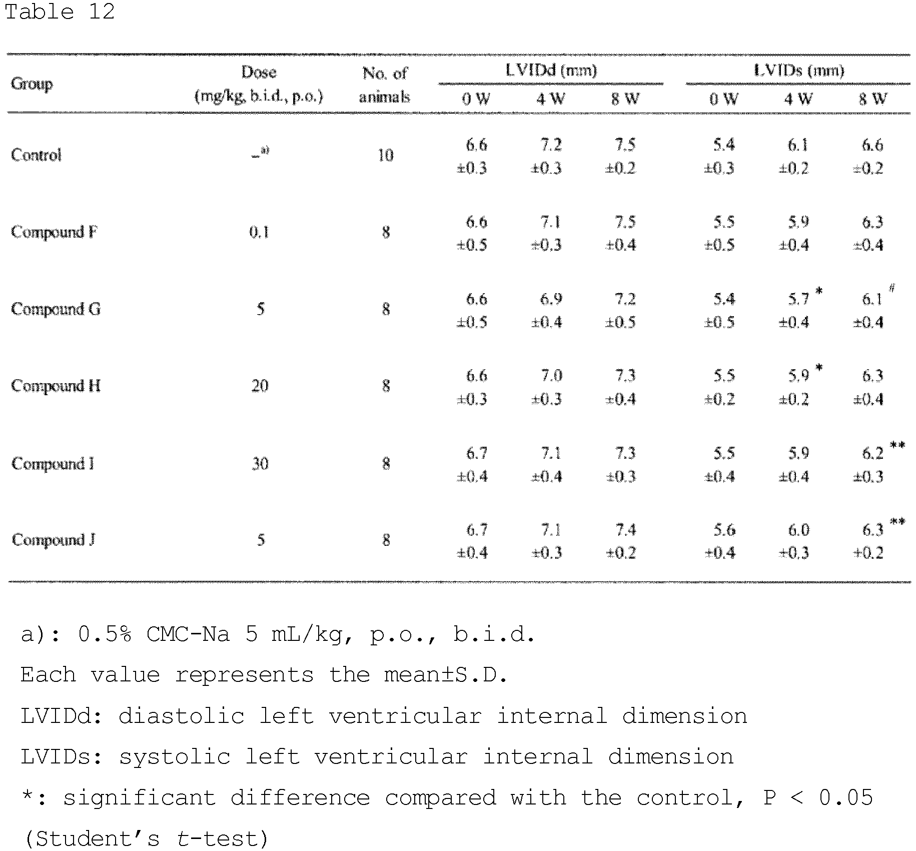

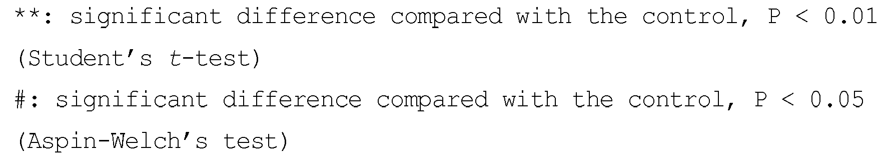

The results of individually orally administering each of the pharmaceutical products repeatedly for 8 weeks twice daily, after the onset of pathological condition, i.e., from the age of 20 weeks until the age of 28 weeks, in the spontaneous dilated cardiomyopathy (J2N-k) hamster model revealed that an action of significantly suppressing deterioration of cardiac function was shown in both EF and FS% in F to J. (J) the lovastatin administration group showed a body weight significantly lower by about 10% than that in the control group; however, all of the other groups, i.e., F to I, showed no change in the body weight, compared with the control group.

### 5) Test 3

In Test 3, the pharmaceutical products selected in Tests 1 and 2 were compared in terms of the clinical dose. Table 13 shows the constitution of the groups.

**Table 13**

| Group | Dose | Period or frequency of administration |
|---|---|---|
| Control | Vehicle (0.5% CMC-Na) 5 mL/kg, p.o., b.i.d. | 8 weeks |
| Test substance C (ozagrel; OZ) | 3.3 mg/kg, p.o., b.i.d. | |
| Test substance D (cilostazol; CS) | 1.7 mg/kg, p.o., b.i.d. | |
| Test substance E (pirfenidone; PF) | 15 mg/kg, p.o., b.i.d. | |
| Test substance G (nintedanib; OB) | 2.5 mg/kg, p.o., b.i.d. | |
| Test substance O (carvedilol; CV) | 0.17 mg/kg, p.o., b.i.d. | |
| Test substance; Preparation 2 (camostat·MS; B·MS) | 10 mg/kg, s.c., once/4 W | Once in 0W and 4W, twice in total |

Reason the doses were set (assuming that the adult body weight was 60 kg, the same amounts as the maximum clinical doses were set)
1) The clinical dose of C (ozagrel; OZ) was 200 mg twice per day. The dose was thus set to 3.3 mg/kg twice per day.
2) The clinical dose of D (cilostazol; CS) was 100 mg twice per day. The dose was thus set to 1.7 mg/kg twice per day.
3) The clinical dose of E (pirfenidone; PF) was 600 mg three times per day, i.e., 1800 mg/day in total. The dose was thus set to 15 mg/kg twice per day.
4) The clinical dose of G (nintedanib sulfonate; OB) was 150 mg twice per day. The dose was thus set to 2.5 mg/kg twice per day.
5) The clinical dose of P (carvedilol; CV) was 10 mg twice per day. The dose was thus set to 0.17 mg/kg twice per day.
6) Preparation 2 (PLGA·MS preparation of camostat; B·MS): the clinical dose of active substance camostat was 200 mg three times per day, i.e., 600 mg/day in total; thus, intermittent subcutaneous administration at a daily dose of 10 mg/kg once every 4 weeks, twice in total was set. The dose was 1/28 of the total oral dose amount.

The dose volume in oral administration was 5 mL/kg (the amount of liquid to be administered to each animal was calculated based on the most recent body weight), and administered by oral gavage using a polypropylene disposable syringe and a rat stomach tube.

The administration period of each pharmaceutical product was 8 weeks.

### Results

### (1) Changes in body weight (Table 14)

Each administration group showed a body weight change similar to that of the control group until 8 weeks after the start of administration of each test substance, and it was confirmed that no side effects occurred.

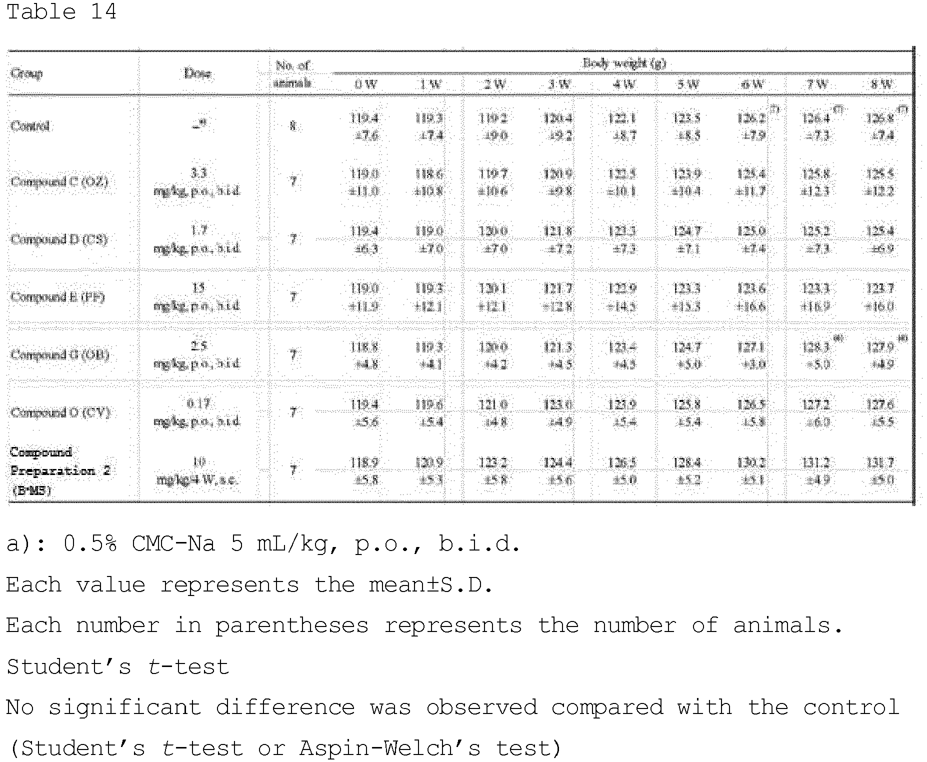

### (2) Cardiac function test: EF value (Table 15) and FS% value (Table 16)

At the time of grouping (0W): No significant difference was observed in any of the administration groups compared with the control group.

4W value: In repeated oral administration of (C) ozagrel, (D) cilostazol, (E) pirfenidone, and (P) carvedilol twice daily; and subcutaneous administration of (B·MS) camostat MS once every 4 weeks, twice in total, a significant cardiac function improvement effect was observed in the actual measured value or the change rate in each group, compared with the control group.

8W value: A significant cardiac function improvement effect was observed in the actual measured value and/or the amount of change in each of (D), (E), (G), and (P), compared with the control group.

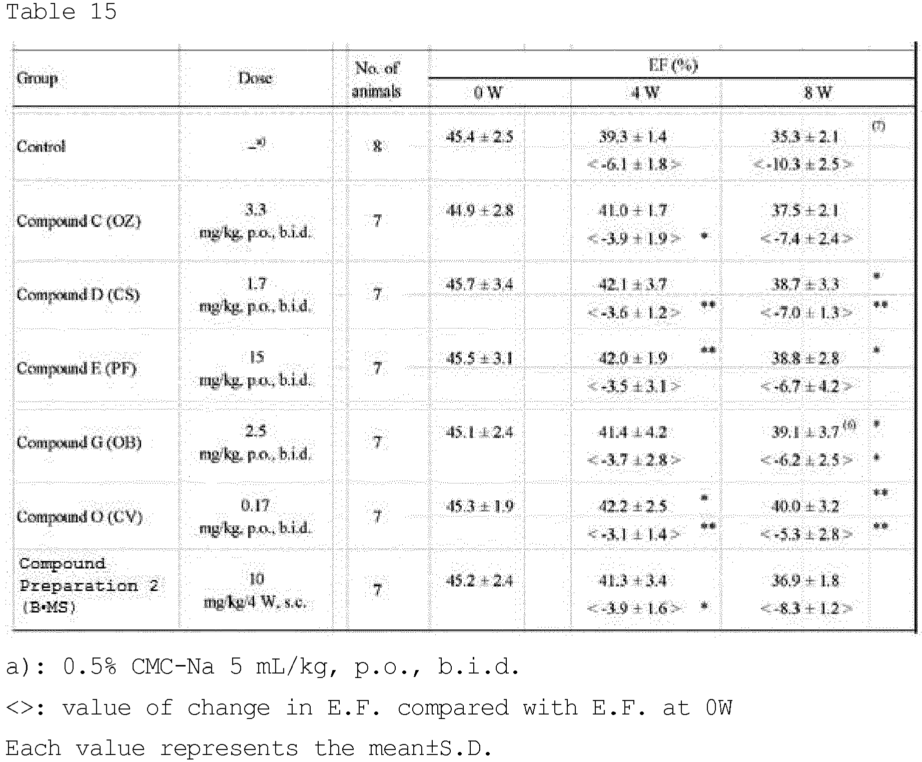

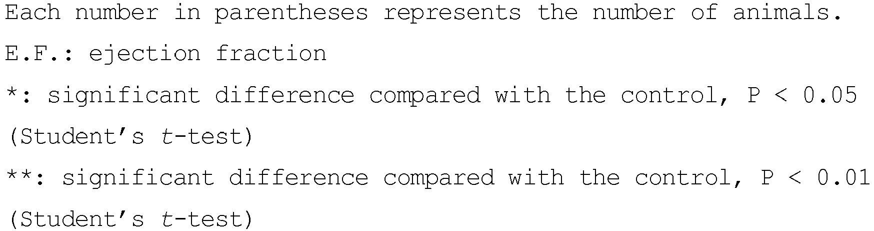

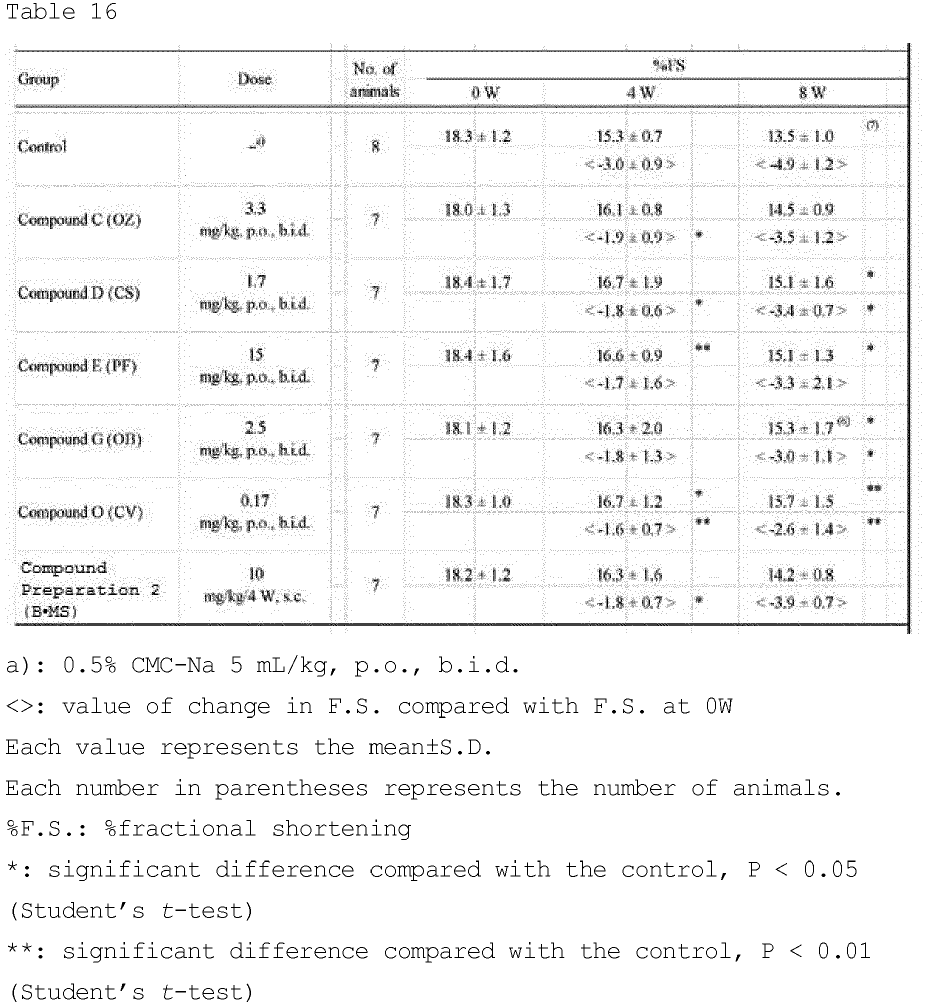

A cardiac function improvement effect was observed in the repeated oral administration of (C) ozagrel, (D) cilostazol, (E) pirfenidone, and (P) carvedilol twice daily; and subcutaneous administration of (B·MS) camostat MS once every four weeks, twice in total.

### 6) Test 4

### Investigation of effects of administration of combination in baby animals

δ-Glycan-deficient spontaneous dilated cardiomyopathy (J2N-k) male hamsters (Japan SLC, Inc.) were delivered at 4 weeks of age. After a 1-week quarantine/acclimation, echocardiography was performed. The hamsters were grouped by stratified random assignment based on the EF values and the body weights so that each administration group was equalized.

Each test substance was individually administered twice daily (at an interval of 8 hours or more) by oral gavage, and echocardiography was performed 4 and 8 weeks after the start of administration (before dissection). Differences from the value before the start of administration (at the time of grouping) (amount of change: Δ) in each group were examined, and the cardiac function (EF value, FS%, etc.) was compared with that in the vehicle group (Group 1). (1) Table 17 shows the constitution of the groups in Test 4.

**Table 17**

| Group | Dose | Period of administration | Number of animals (animal no.) |
|---|---|---|---|
| Control | Vehicle (0.5% CMC-Na) 5 mL/kg, p.o., b.i.d. | 8 weeks | 8 (101-108) |
| Test substance O (CV) | 0.17 mg/kg, p.o., b.i.d. | | 7 (201-207) |
| Test substance C (OZ) | 3.3 mg/kg, p.o., b.i.d. | | 7 (301-307) |
| Test substance E (PF) | 15 mg/kg, p.o., b.i.d. | | 7 (401-407) |
| Test substances O+C (CV+OZ) | 0.17 mg/kg + 3.3 mg/kg, p.o., b.i.d. | | 7 (501-507) |
| Test substances O+E (CV+PF) | 0.17 mg/kg + 15 mg/kg, p.o., b.i.d. | | 7 (601-607) |
| Test substances O+C+E (CV+OZ+PF) | 0.17 mg/kg + 3.3 mg/kg + 15 mg/kg, p.o., b.i.d. | | 7 (701-707) |

In Test 4, comparisons were made of effects of three pharmaceutical products, O (CV; carvedilol), C (OZ; ozagrel), and E (PF; pirfenidone), selected in Tests 1, 2, and 3, alone or in combination at the clinical doses.

The doses were set according to the dose-setting basis in Test 3, and similarly orally administered twice daily. Effects of administration of combinations of two or three of carvedilol, which is a β-blocker widely used clinically, and ozagrel and/or pirfenidone were examined.

### Results

### (1) Changes in body weight (Table 18)

Each administration group showed a body weight change similar to that of the control group until 8 weeks after the start of administration of each test substance, and it was confirmed that no side effects occurred.

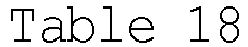

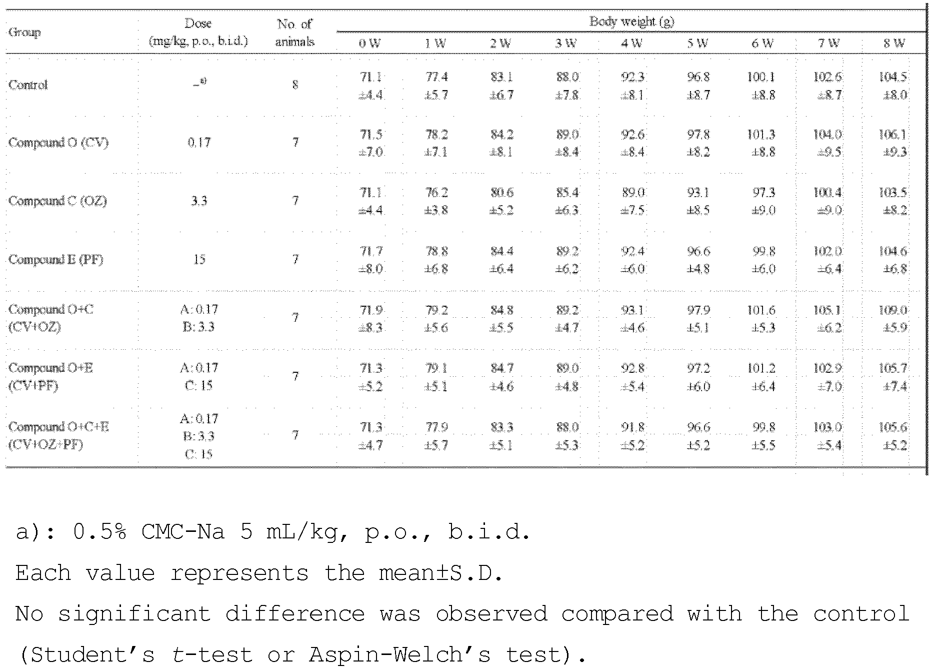

### Cardiac function test: EF value (Table 19) and FS% (Table 20)

A significant suppression action on a decrease in left ventricular systolic function was also observed in administration of carvedilol alone and ozagrel hydrochloride hydrate alone at the clinical doses. Further, administration of a combination of two drugs, carvedilol and ozagrel hydrochloride hydrate, enhanced the action; and administration of three drugs, carvedilol, ozagrel hydrochloride hydrate, and pirfenidone, provided further synergistic action of suppressing a decrease in left ventricular systolic function.

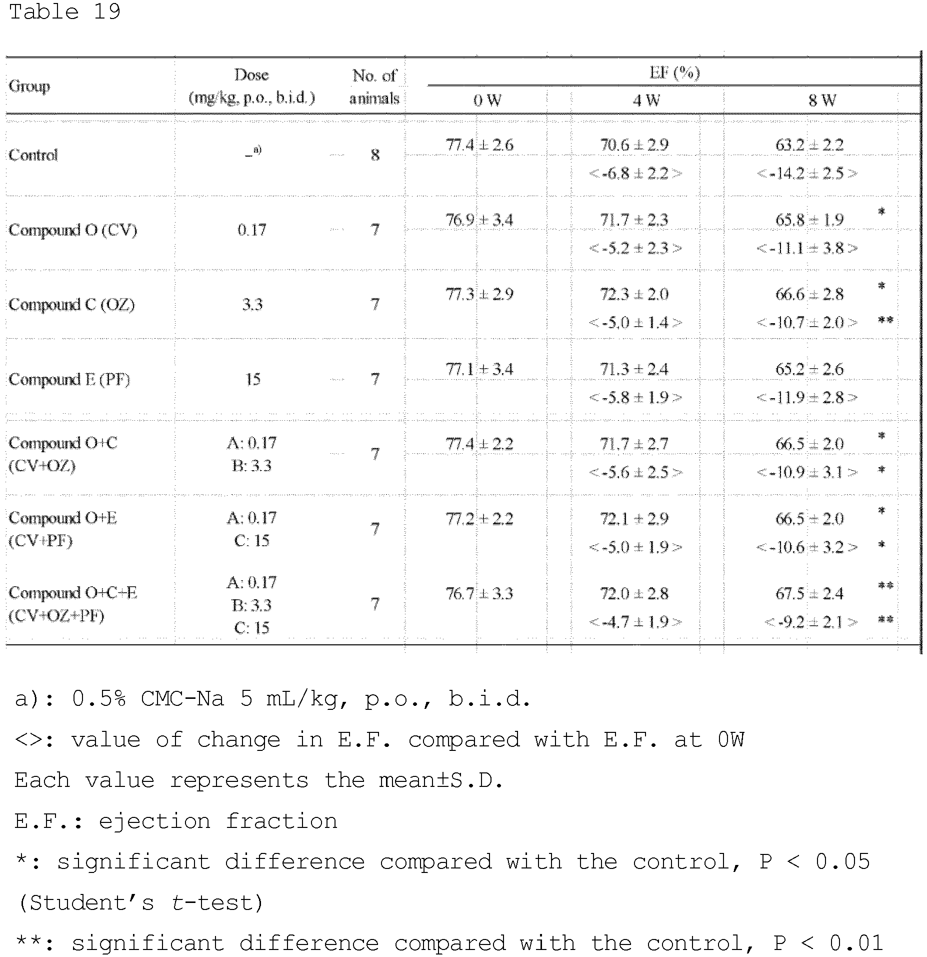

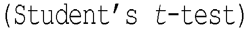

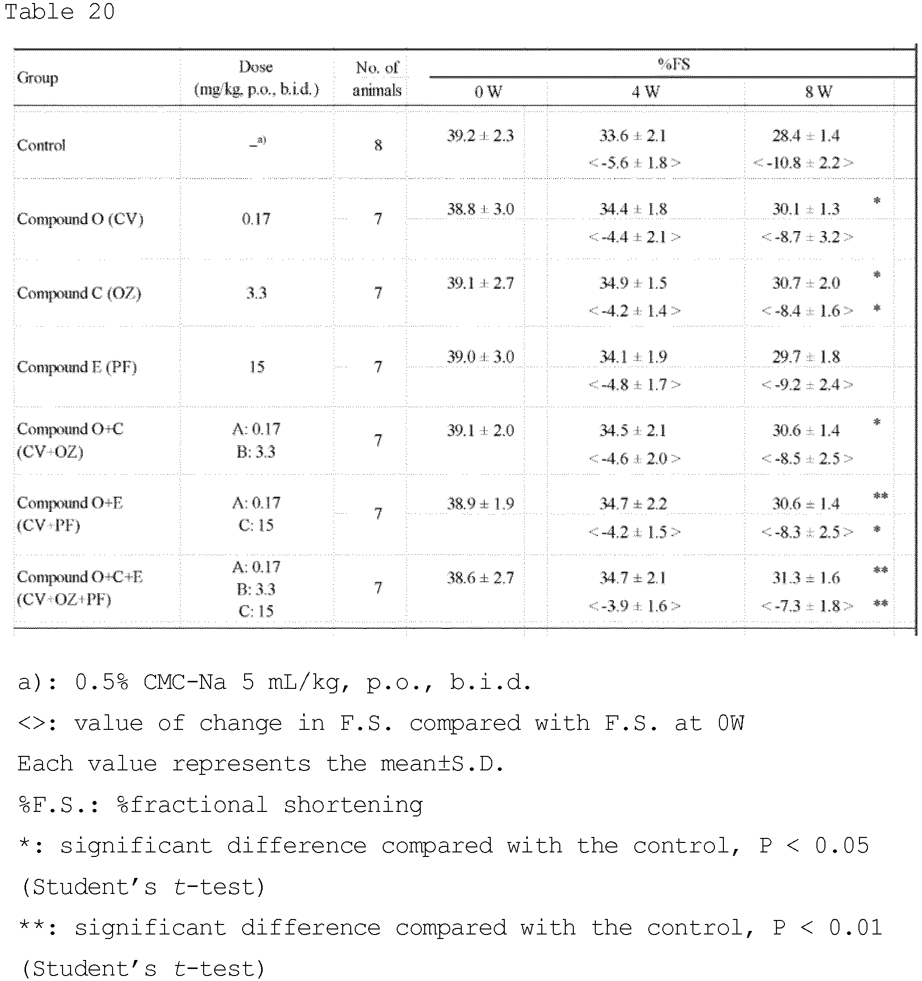

The above results revealed that administration of (O) carvedilol alone and (C) ozagrel hydrochloride hydrate alone to the spontaneous dilated cardiomyopathy J2N-k baby hamsters showed an action of significantly suppressing a decrease in left ventricular systolic function, even at the clinical doses; and was confirmed to show a suppression effect on aggravation of cardiomyopathy. Furthermore, administration of three drugs, (O) carvedilol, (C) ozagrel hydrochloride hydrate, and (E) pirfenidone, in combination, showed a synergistic action of suppressing a decrease in left ventricular systolic function.

As a result of administration based on the no-observed-adverse-effect levels determined from the toxicity study, it was confirmed that all of A to J are useful in suppressing deterioration of cardiac function for dilated cardiomyopathy.

### 2. Investigation of effects on model of ischemia (MI) by rat coronary artery complete ligation

A rat coronary artery complete ischemia model was prepared, and changes in cardiac function by administration of each test substance were compared.

A coronary artery complete ischemia model was prepared by completely occluding the left anterior descending coronary artery (LAD) of rats. Each test substance was orally administered individually twice daily from the day following the preparation of the model (after 24 hours), and measurement of body weight and echocardiography were performed 1, 2, and 4 weeks after the start of administration. The heart was removed 4 weeks after the start of administration, and electron microscopy and RNA/protein measurement were performed. Specimens for immunopathology were also collected and evaluated.

### (1) Preparation of myocardial ischemia model

Rats were anesthetized with pentobarbital sodium (Somnopentyl (Kyoritsu Seiyaku Corporation): 35 to 45 mg/kg, i.p.). After anesthesia, the rats were fixed in the supine position. A tracheal tube was inserted through the mouth into the respiratory tract; and the rats were ventilated with a respirator for small animals (Model 683, Harvard Apparatus, Inc.) (tidal volume: 1.5 to 2.0 mL/stroke and frequency of breath: 70 strokes/min), and subjected to thoracotomy at the chest sidewall to expose the heart. The left anterior descending coronary artery (LAD) was completely occluded using a surgical needle with a thread (ELP; ELP surgical needle with a thread: M10-60B2). At this time, an electrocardiogram (Lead II; however, if measurement was difficult, Lead aVR) was measured via an electrocardiogram amplifier (AC-601G; Nihon Kohden Corporation); and whether there were increases in ST electric potential and whitening of cardiac muscle was observed macroscopically to confirm the presence or absence of occlusion (onset of myocardial ischemia). If ventricular fibrillation (VF) occurred, resuscitation treatment was performed by directly stimulating the heart using ring forceps; and if VF disappeared, such a rat was used. Thereafter, the chest was closed, and the incision site was sutured and cleaned with an isodine liquid for animals (Meiji Seika Pharma Co., Ltd.). The day following the preparation of the coronary artery complete ischemia model was regarded as 1 day after.

### (2) Grouping

Rats that did not show, in general symptom observation, weakening (reduced locomotor activity, respiratory distress, pallor of pinna (decreased body temperature), etc.) due to pathological condition on the day following the preparation of the coronary artery complete ischemia model, were selected. On the day following complete ligation of the coronary arteries, echocardiography was performed, and animals whose ejection fraction (EF) decreased by 25% or more from that of normal animals (EF 90%) were selected. The rats were grouped by stratified random assignment based on the ejection fractions (EF) in echocardiography and the body weights so that each administration group was equalized.

### (3) Echocardiography

The rats were fixed in the supine position under 2.0% isoflurane (isoflurane inhalation anesthetic solution; Pfizer) anesthesia using an inhalation anesthesia apparatus for experimental animals (TK-5; Biomachinery) and an anesthesia apparatus for small animals (MK-A110S; Muromachi Kikai Co., Ltd.), and echocardiographic measurement was performed using a ultrasonic diagnostic imaging apparatus (Nemio SSA-550A; Toshiba Medical Systems Corporation). A linear probe (14 MHz) was placed on the chest of the rats to calculate the fractional shortening [%FS = (LVIDd-LVIDs) × 100/LVIDd] and the ejection fraction [EF = (LVIDd3-LVIDs3)/LVIDd3]. The measurement was carried out for 3 heartbeats per image, and the average values thereof were used as the measured values.

The measurement was performed 4 times, i.e., on the day following the complete ligation of the coronary arteries (at the time of grouping; Day 1), 7 days after the complete ligation of the coronary arteries (before the first administration on Day 7 after the start of administration; Day 7), 14 days after the complete ligation of the coronary arteries (before the first administration on Day 14 after the start of administration; Day 14), and 29 days after the complete ligation of the coronary arteries (before dissection on the day after Day 28 after the start of administration, which was the final day of administration; Day 29).

After the final body weight measurement and completion of echocardiography, about 3 mL of blood was collected from the abdominal aorta under isoflurane anesthesia using a disposable syringe (Nipro syringe). The blood was treated with heparin (Venoject vacuum blood collection tube), and centrifuged with a high-speed cooling centrifuge (Model 6000; Kubota Corporation Co., Ltd.) (3000 rpm, 4°C, 10 min) to collect plasma.

After the completion of blood collection, the rats were euthanized by exsanguination, and the heart was removed. The weight of the heart was measured; the infarct area, including the left ventricle and the right ventricle, were then divided into three parts along the short axis; and photographs of short-axis cross-sections were taken. Specifically, the apical portion was removed, and the middle area was sectioned into two parts at an interval of about 2 mm. Thereafter, the two sections on the apical and basal sides, one per side, were photographed and recorded. After photography, the apical side (bottom portion) and the basal side (top portion) were stored in buffered formalin. Moreover, one section of a peri-infarct site was collected from the middle portion, immersed in RNAlater, and stored in a refrigerator (5°C) overnight. On the following day, the RNAlater was removed, and the section was then frozen as is in liquid nitrogen and stored frozen at -64.5°C or less (ultra-low-temperature bath (CLN-35C; Nihon Freezer Co., Ltd.); set temperature: -80°C). The remainder of the middle portion was stored frozen as is.

### 1) Test 1

### Test of MI model in administration at no-observed-adverse-effect level (NOAEL)

Table 21 shows the constitution of the groups in Test 1.

**Table 21**

| Group | Test substance | Dose (twice per day)* | Number of animals |
|---|---|---|---|
| 1 | Control (vehicle) | - | 8 |
| 2 | A (ONO-1301) | 3 mg/kg | 8 |
| 3 | F (Beraprost) | 0.1 mg/kg | 8 |
| 4 | C (Ozagrel) | 50 mg/kg | 8 |
| 5 | F (Beraprost) + C (Ozagrel) | 0.1 mg/kg + 50 mg/kg | 8 |
| 6 | D (Cilostazol) | 30 mg/kg | 8 |
| 7 | E (Pirfenidone) | 50 mg/kg | 8 |

| | | | |
|---|---|---|---|
| *: 28-day repeated oral administration was performed at the doses described above twice per day. The interval between the two daily doses was 8 hours or more. | | | |

### Results

### (1) Changes in body weight (Table 22)

Each of (A) the ONO-1301 administration group, (C) the ozagrel hydrochloride hydrate administration group, the administration group of (F) beraprost + (C) ozagrel hydrochloride hydrate, (D) the cilostazol administration group, and (E) the pirfenidone administration group showed a body weight change similar to that of the control group until Day 29.

Significant body weight gain suppression was observed in (F) the beraprost administration group over a period of Day 14 to Day 29, compared with the control group (P < 0.05 vs. Control group in each). Although significant changes in the body weight were observed (two points in time in measurement), they were slight as the amount of change, and were considered to be of no biological significance.

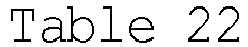

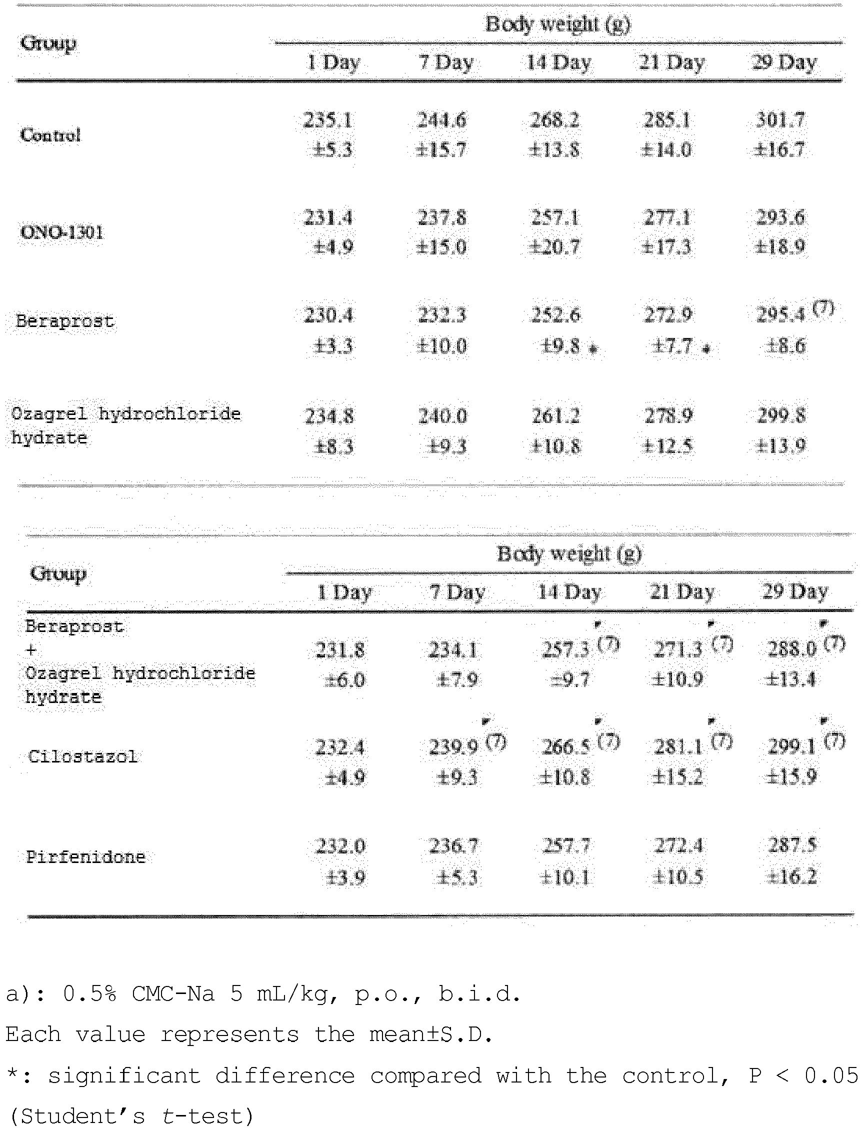

### (2) Cardiac function test (EF value) (Table 13)

At the time of grouping (Day 1), no significant difference was observed in any of the administration groups compared with the control group.

On Day 7, the actual measured value was significantly higher in the cilostazol administration group (P < 0.05 vs Cont group) and each of the administration groups other than the cilostazol administration group (P < 0.01 vs Cont group).

The amount of change was not significantly higher in only the cilostazol administration group; however, the amount of change was significantly higher in each of the administration groups other than the cilostazol administration group (P < 0.05 vs Cont group).

On Day 14, both the actual measured value and the amount of change were significantly higher in each administration group, than in the control group (P < 0.01 vs Cont group).

On Day 29 (final day), both the actual measured value and the amount of change were significantly higher in each administration group, than in the control group (P < 0.01 vs Cont group).

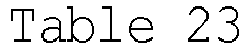

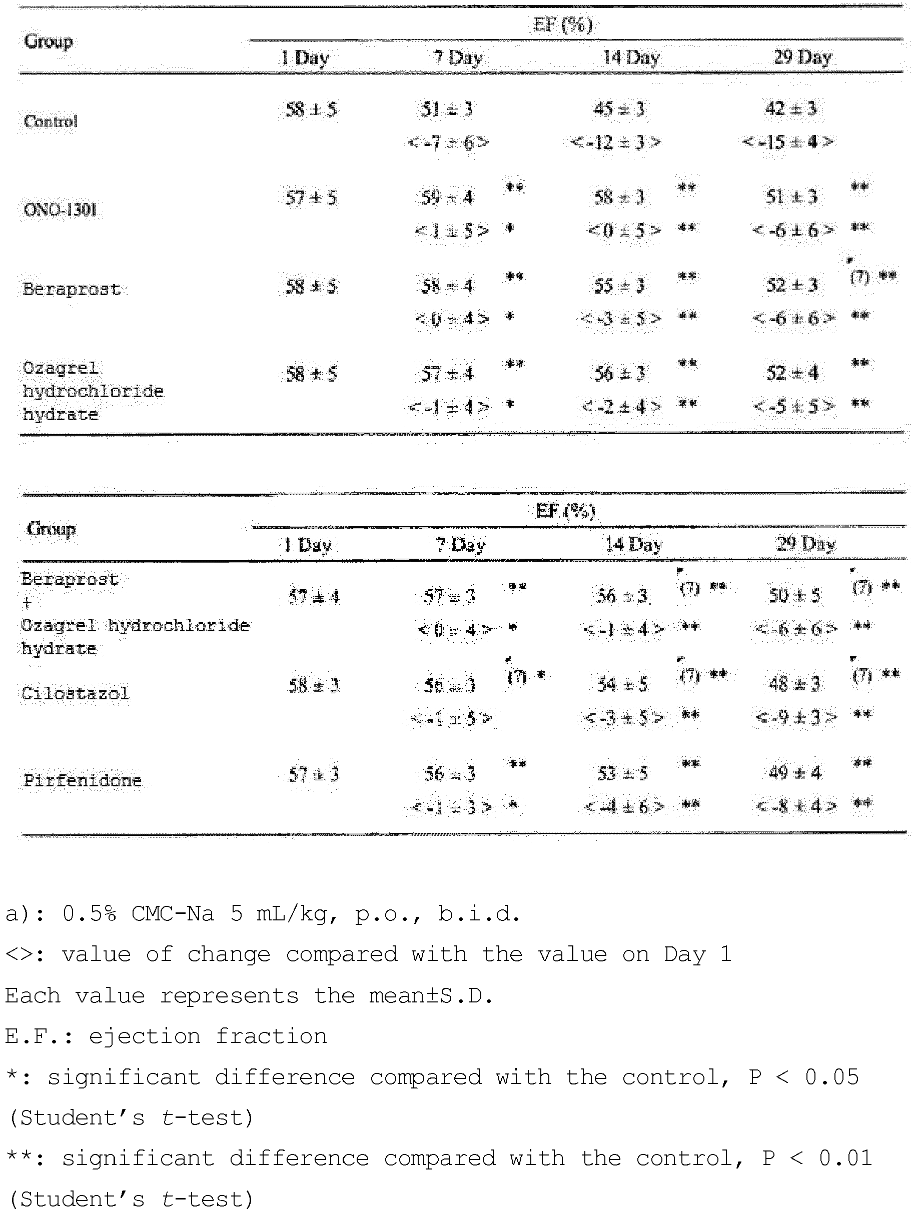

### (3) Cardiac function test (%FS) (Table 14)

At the time of grouping (Day 1), no significant difference was observed in any of the administration groups compared with the control group.

On Day 7, the actual measured EF value was significantly higher in the cilostazol administration group (P < 0.05 vs Cont group) and each of the administration groups other than the cilostazol administration group (P<0.01 vs Cont group). The amount of change was not significantly higher in only the cilostazol administration group; however, the amount of change in the EF value was significantly higher in each of the administration groups other than the cilostazol administration group (P < 0.05 vs Cont group).

On Day 14, both the actual measured EF value and the amount of change in the EF value were significantly higher in each administration group, than in the control group (P < 0.01 vs Cont group).

On Day 29, the actual measured value was significantly higher in each administration group, than in the control group (P < 0.01 vs Cont group). The amount of change was significantly higher in the cilostazol administration group (P < 0.05 vs Cont group) and each of the administration groups other than the cilostazol administration group (P < 0.01 vs Cont group), than in the control group.

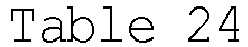

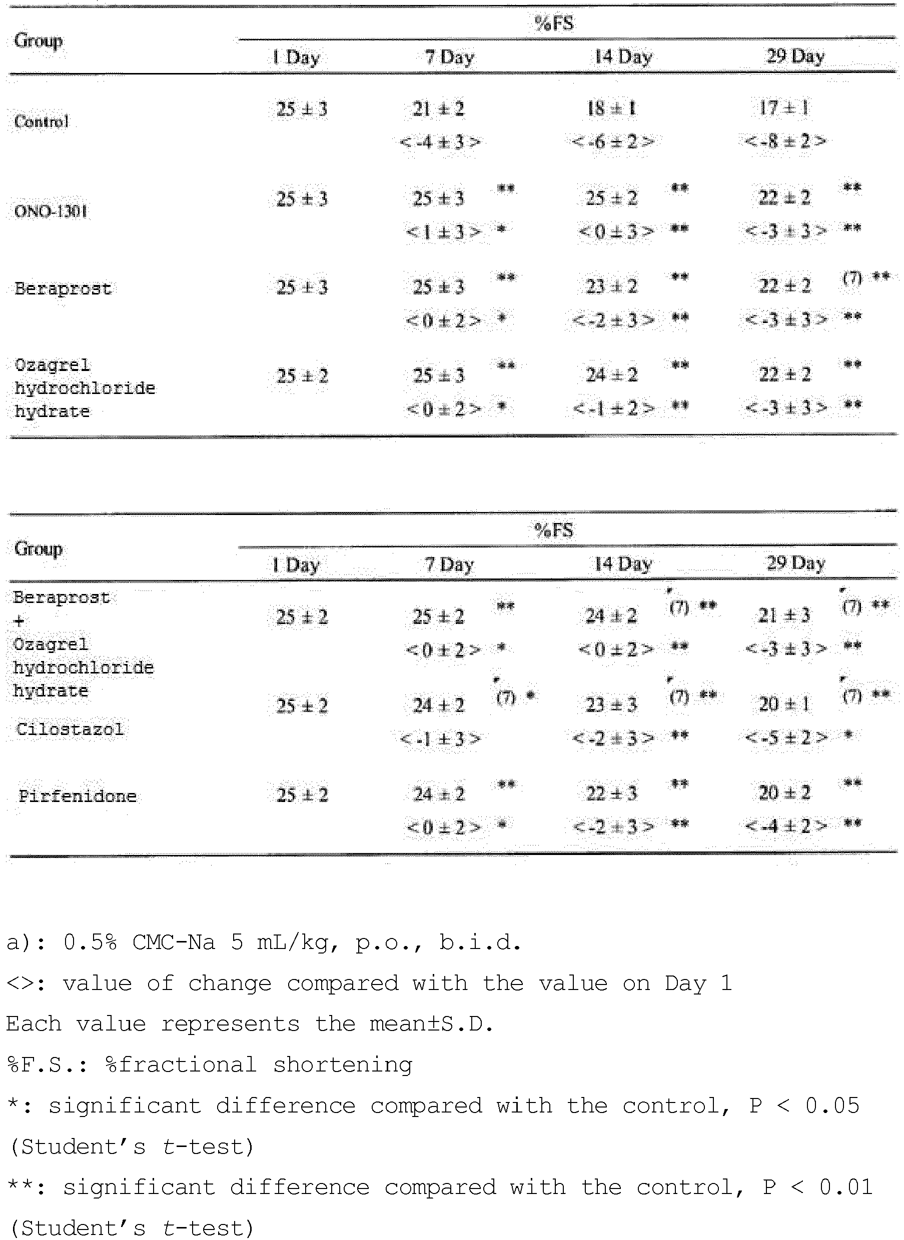

The above results confirmed that the coronary artery complete ischemia model prepared in this test (control group) is a myocardial infarction model, since left ventricular systolic dysfunction (decrease in ejection fraction (EF); decrease in fractional shortening (%FS)) was observed.

The test substance A (ONO-1301), the test substance F (beraprost), the test substance C (ozagrel hydrochloride hydrate), the test substances F + C (beraprost + ozagrel hydrochloride hydrate), the test substance D (cilostazol), and the test substance E (pirfenidone) exhibited a suppression action on the deterioration of left ventricular systolic function, and showed an action of improving myocardial infarction.

Administration of a combination of the test substance F (beraprost) and the test substance C (ozagrel hydrochloride hydrate) significantly improved left ventricular systolic function compared with the control group. However, effect enhancement was not observed by administration of the combination of F and C compared with administration of F alone and C alone, suggesting that administration of F alone and C alone already exerted their nearly maximum effects in this test system.

### 2) Test 2

Effects of repeated oral administration of B, G, I, and C and single subcutaneous administration of sustained-release preparations of B, C, and I (Preparation 1 to Preparation 3) were investigated.

### (1) Table 25 shows the constitution of the groups.

**Table 25**

| Group | Test substance | Dose | Administration route | Number of animals |
|---|---|---|---|---|
| 1 | Control (vehicle) | - | Oral | 6 |
| 2 | B (Camostat) | 150 mg/kg | Oral | 5 |
| 3 | G (Ofev) | 5 mg/kg | Oral | 6 |
| 4 | I (Sildenafil) | 30 mg/kg | Oral | 5 |
| 5 | C (Ozagrel) | 10 mg/kg | Oral | 5 |
| 6 | B·MS (Preparation 2; camostat·MS) | 100 mg/kg | Single subcutaneous | 5 |
| 7 | C·MS (Preparation 1; ozagrel·MS) | 50 mg/kg | Single subcutaneous | 6 |
| 8 | I·MS (Preparation 3; sildenafil·MS) | 30 mg/kg | Single subcutaneous | 6 |

Oral administration groups: 28-day repeated oral administration was performed at the doses described above, twice per day. The interval between the two daily doses was 8 hours or more. To the control group, vehicle (CMC-Na) was orally administered. Subcutaneous administration groups: single subcutaneous administration at each dose was performed 24 hours after complete ligation of the coronary arteries.

### Dose-setting basis

### Reasons the doses were set

The dose of each test substance was the maximally tolerated dose set based on the no-observed-adverse-effect level.

### B (camostat mesilate)

Since suppression of body weight gain was observed at 550 mg/kg or more in repeated oral administration in rats for 6 months, the maximum no-observed-adverse-effect level was 235 to 550 mg/kg. The maximally tolerated dose was thus set to 150 mg/kg.

### G (nintedanib ethanesulfonate)

Side effects developed at 20 mg/kg/day in a 6-month repeated administration toxicity study in rats, and the no-observed-adverse-effect level was 5 mg/kg. The maximum dose was thus set to 5 mg/kg twice per day.

### I (sildenafil citrate)

As a result of oral administration of sildenafil to SD rats for 6 months, side effects were observed in the 60 mg/kg group. The no-observed-adverse-effect level was 60 mg/kg/day. The maximum dose was thus set to 30 mg/kg twice per day.

### C (ozagrel hydrochloride hydrate)

Side effects were observed at 500 mg/kg or more in repeated oral administration in rats for 3 months, and the no-observed-effect level was 150 mg/kg. The no-observed-adverse-effect level was thus set to 50 mg/kg in 1. Since a sufficient effect was confirmed at 50 mg/kg in Test 1, the dose was set to 10 mg/kg, which is close to the clinical dose (6.7 mg/kg), in Test 2.

### B·MS (Preparation 2; camostat·MS)

Since the LD₅₀ in subcutaneous administration of camostat in rats was 1329 mg/kg, the dose was set to about 1/10 thereof, i.e., 100 mg/kg. The daily dose in Group 2 (test substance B) in Test 1 for hamsters was 300 mg/kg (150 mg/kg × 2). The above dose was 1/3 thereof (total dose amount ratio: 150×2×28/100, i.e., 1/84).

### C·MS (Preparation 1; ozagrel hydrochloride·MS)

Since the LD₅₀ in subcutaneous administration of ozagrel in rats was 2049 mg/kg, the dose was set to 1/40 thereof, i.e., 50 mg/kg. Efficacy was confirmed in administration at 50 mg/kg, twice per day, in Group 4 in Test 1 for hamsters. The above dose was a dose thereof, i.e., 50 mg/kg. The daily dose in Group 5 (test substance L) in this test was 20 mg/kg (10 mg/kg twice per day) (total dose amount: 10×2×28/50, i.e., 1/11.2).

### I·MS (Preparation 3; sildenafil·MS)

The lethal dose of sildenafil was 10 mg/kg or more in intravenous administration in rats, and 1000 mg/kg or more in oral administration. The dose was thus set to 30 mg/kg. Efficacy was confirmed in administration at 30 mg/kg, twice per day, in Group 5 in Test 2 for hamsters. Thus, the above dose was a dose thereof, i.e., 30 mg/kg. The dose in Group 4 (test substance I) in this test was 30 mg/kg twice daily (total dose amount: 30×2×28/30, i.e., 1/56).

### Results

### (1) Changes in body weight (Table 26)

Each of the B (camostat) administration group, the G (Ofev) administration group, the I (sildenafil) administration group, the C (ozagrel) administration group, the B·MS (camostat·MS) administration group, the C·MS (ozagrel·MS) administration group, and the I·MS (sildenafil·MS) administration group showed a body weight change similar to that of the control group until Day 29.

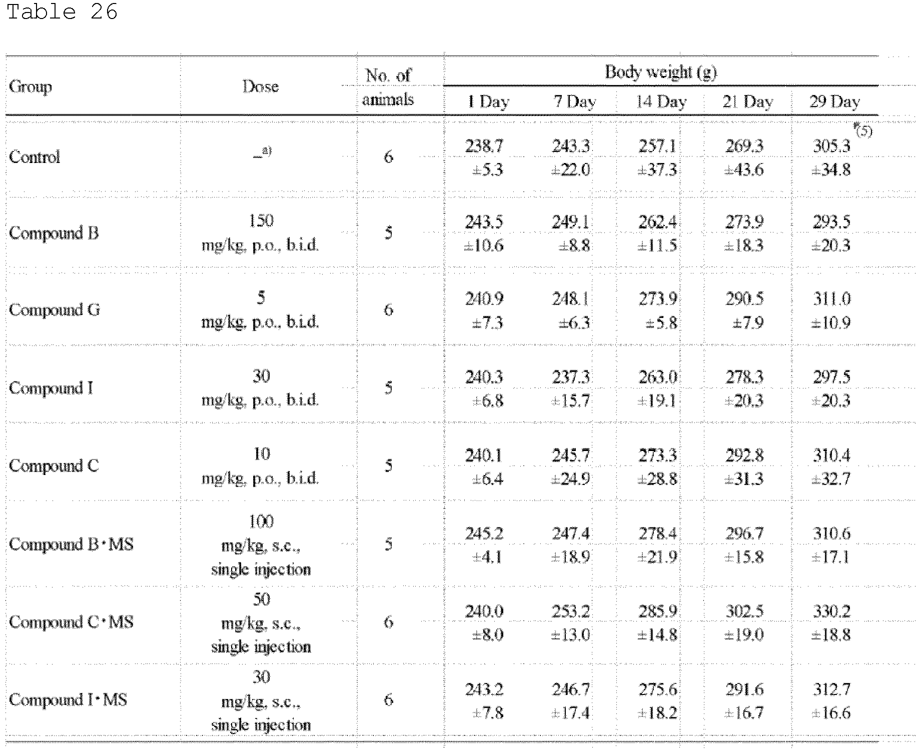

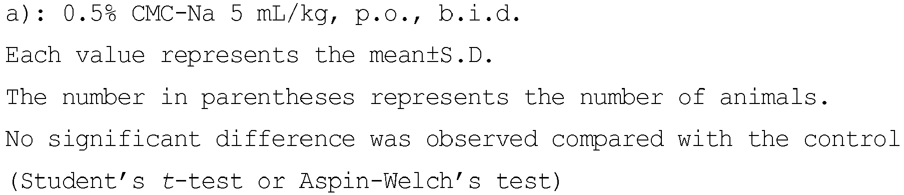

### (2) Cardiac function test (EF value) (Table 27)

At the time of grouping (Day 1), no significant difference was observed in any of the administration groups compared with the control group. On both Day 14 and Day 29, the actual measured value and/or the amount of change was significantly higher in all of the twice-daily repeated oral administration groups of B, G, I, and C, than in the control group. Moreover, the actual measured value and/or the amount of change was significantly higher in single subcutaneous administration of B·MS (Preparation 2), C·MS (Preparation 1), and I·MS (Preparation 3), than in the control group, on both Day 14 and Day 29.

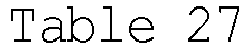

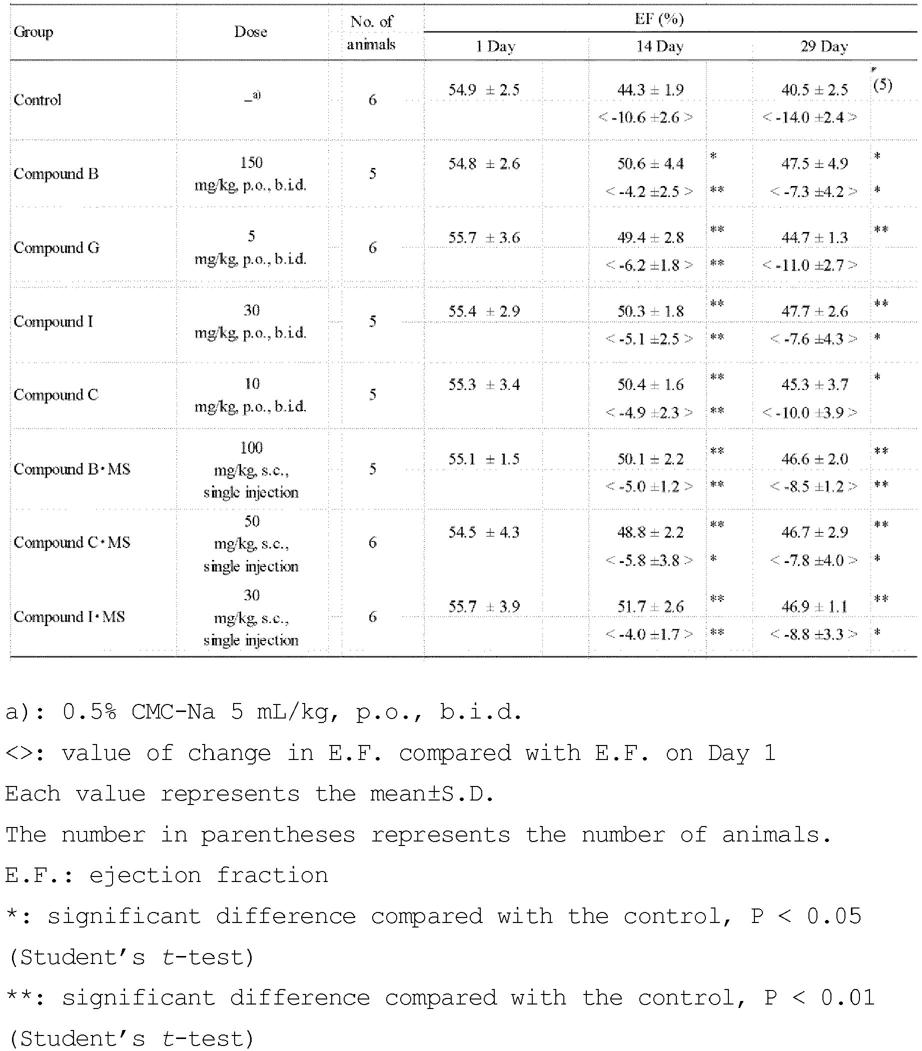

### (3) Cardiac function test (%FS) (Table 28)

At the time of grouping (Day 1), no significant difference was observed in any of the administration groups compared with the control group. On both Day 14 and Day 29, the actual measured value and/or the amount of change was significantly higher in all of the test substance administration groups as in EF, than in the control group.

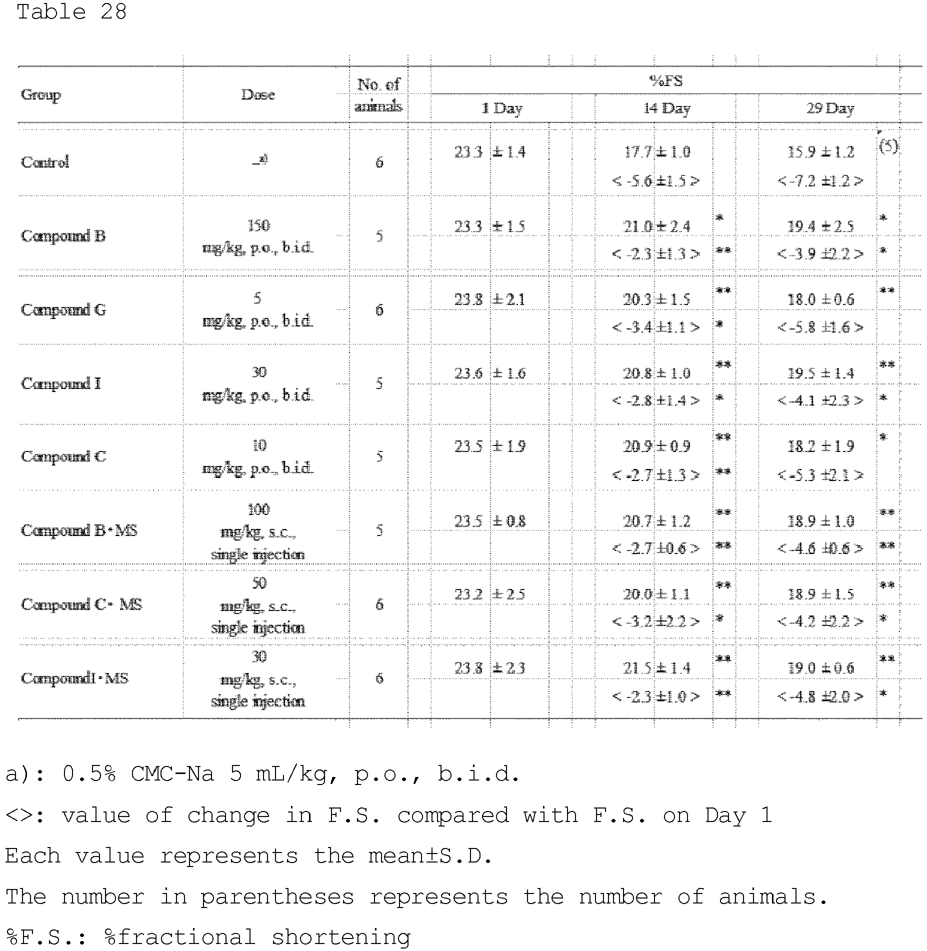

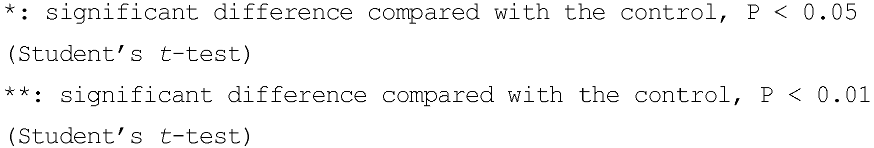

The values of cardiac function (EF, FS%) were significantly higher in all of the groups of B, G, I, and C repeatedly orally administered twice daily for 4 weeks and the single subcutaneous administration groups of B·MS (Preparation 29), C·MS (Preparation 1), and I·MS (Preparation 3), than in the control group. In particular, it is ground-breaking that efficacy was observed in B·MS at a dose that was 1/84 of the total dose amount of B, in C·MS at a dose that was 1/11.2 of the total dose amount of C, and in I·MS at a dose that was 1/56 of the total dose amount of I.

### 3. Investigation of long-term effects in spontaneous dilated cardiomyopathy (J2N-k) hamster model by comparison

ONO-1301 and (K) candesartan, which is ARB, were each individually orally administered to a δ-glycan-deficient spontaneous dilated cardiomyopathy (J2N-k) hamster model twice daily repeatedly for 36 weeks, after the onset of pathological condition (20 weeks old); and changes in cardiac function and survival rates in long-term administration were compared for evaluation. Specifically, echocardiographic measurement was performed after the onset of pathological condition (20 weeks old); the animals were grouped evenly based on the ejection fractions (EF values) and the body weights (Table 15), and each test substance was individually administered for 36 weeks.

The test substances, i.e., (A) ONO-1301 and (K) candesartan, which is ARB, were compared.

The test was performed in the same manner as described in the section "1. Investigation of effects in spontaneous dilated cardiomyopathy (J2N-k) hamster model" above.

**Table 29**

| | Test group | Administration | Number of animals |
|---|---|---|---|
| 1 | Control | Vehicle twice/day | 10 |
| 2 | (A) ONO-1301 3.0 mg/kg | ONO-1301-0.6 mg/mL twice/day | 10 |
| 3 | (K) Candesartan 3.0 mg/kg | - Candesartan 0.6 mg/mL (first) | 10 |
| | | - Vehicle (second) | |

ONO-1301 was administered at 3 mg/kg twice per day. Candesartan was administered at 3 mg/kg once per day, and only vehicle was administered at the second administration. To the control group, vehicle (0.5% CMC-Na) was administered twice daily.

### (2) Cardiac function test (EF value) (Table 16)

A significant improvement effect was observed in the ONO-1301-3.0 mg/kg group compared with the control group during the administration period. In contrast, although an effect similar to that in the ONO-1301-3.0 mg/kg group was observed in the candesartan-3.0 mg/kg group until 20 weeks after the start of administration, the effect was attenuated thereafter, and the difference was no longer significant in Week 30 and Week 36 (final).

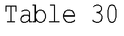

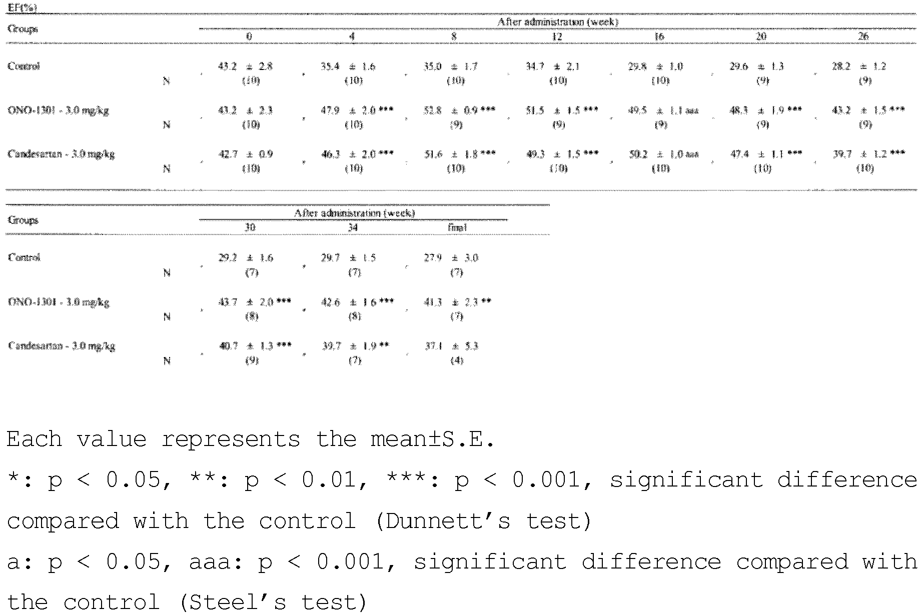

### (3) Cardiac function test (FS%) (Table 17)

Significantly higher values were observed in the ONO-1301-3.0 mg/kg group 1, 2, 3, 4, 5, 6, 7, and 8 months (34 and 36 weeks) after the start of administration and in the candesartan-3.0 mg/kg group 1, 2, 3, 4, 5, 6, 7, and 8 months (34 weeks) after the start of administration, compared with the control group; however, the difference was no longer significant in Week 36 (final) in the candesartan group.

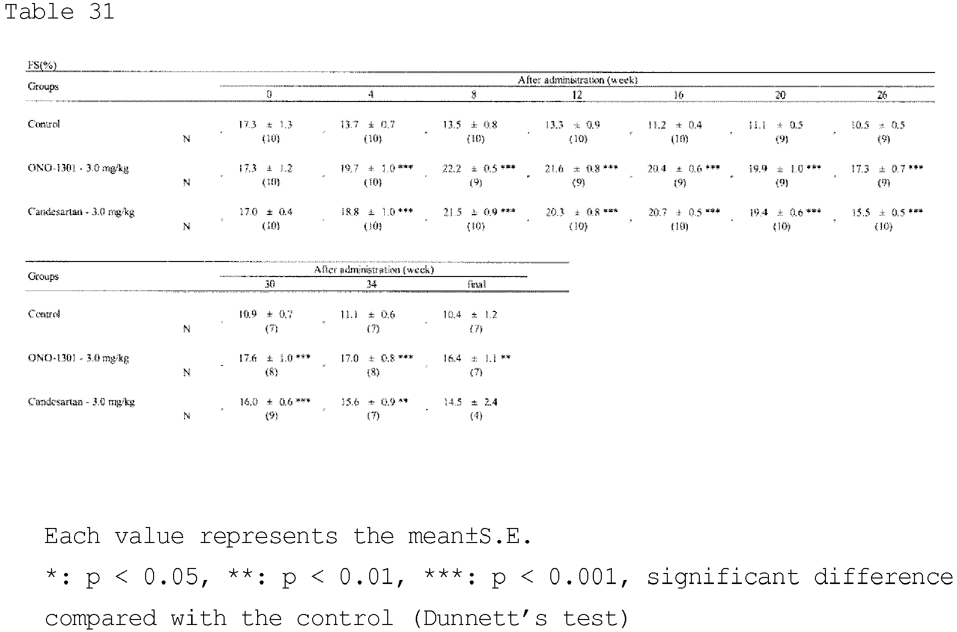

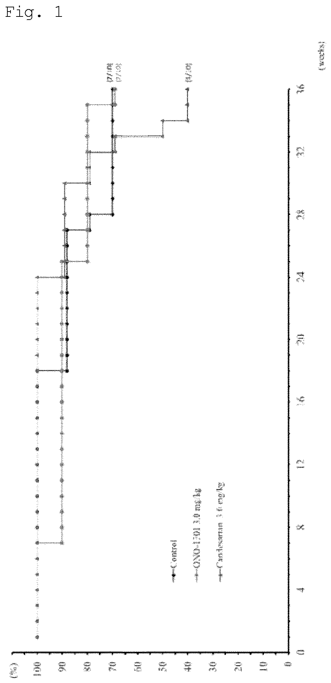

### (4) Survival rate (Fig. 1)

No significant difference in the survival rate was observed between the test substance administration groups and the control group, because the mortality in the Cont group was low. However, in the candesartan group, a rapid decrease in the survival rate was observed from 29 weeks after the start of administration of the test substance, and a difference in the survival rate between the candesartan group and the ONO-1301-3.0 mg/kg group was confirmed.

In echocardiographic measurement of left ventricular function, an improvement effect was observed in the EF (ejection fraction) value, which is used as an indicator of heart failure, in the ONO-1301-3.0 mg/kg group. In the candesartan (control substance; ARB)-3.0 mg/kg group, a similar improvement effect was also observed until about 20 weeks after the start of administration; however, the cardiac function started to decline from about 5 months after the start of administration, and the number of deaths increased in the final phase.

The above results confirmed that ONO-1301 also has an effect superior to that of candesartan, which is generally used for dilated cardiomyopathy as an antihypertensive agent, in long-term administration.

From the above, it was confirmed that ONO-1301 suppresses a transition from dilated cardiomyopathy to heart failure, i.e., deterioration of cardiac function, and suppresses mortality, over a long period of time compared with candesartan (ARB), which is currently used as a first-line drug for dilated cardiomyopathy.

### 4. Investigation of effects of long-term repeated oral administration in canine rapid pacing severe dilated cardiomyopathy model

This test was performed to investigate the development of a versatile cardiovascular/myocardial regeneration therapeutic agent aimed at suppressing aggravation of DCM; and delaying or avoiding heart transplant or implantation of an artificial heart by orally administering ONO-1301 to patients with dilated cardiomyopathy repeatedly for a long period of time, as early therapeutic intervention.

Specifically, the cardiac function improvement effect and survival prolongation effect of long-term repeated oral administration of ONO-1301 were investigated using a canine rapid pacing severe dilated cardiomyopathy model.

### 1) Preparation of rapid pacing-induced canine heart failure model and administration of test substance

### (1) Pacemaker implantation surgery

The right neck of the animals was incised under anesthesia, and an intracorporeal cardiac pacemaker for animals (hereinafter referred to as a "pacemaker," SIP-501; Starmedical, Inc.) was subcutaneously implanted. A retractable screw-in lead (Tendril™ STS 2088TC; 58 cm 6Fr; St. Jude Medical) was inserted from the right jugular vein using a fluoroscope, and its tip was placed on the right ventricular wall. A sham operation was performed for a Sham group. After the pacemaker was operated, and it was confirmed in an electrocardiogram (Lead II) that the heartbeat was linked to the pulse rate, the incision site was sutured.

### (2) Rapid pacing and administration of test substance

A canine model of severe dilated cardiomyopathy induced by rapid pacing (number of beats: 226 to 240 beats/min) was used. The animals were grouped evenly based on the cardiac function (EF) 4 weeks after rapid pacing. ONO-1301 (3 mg/kg) was orally administered twice daily repeatedly for 6 months (26 weeks) (30 weeks after the preparation of the model), and effects of the administration on long-term efficacy (survival rate) were investigated. The dose of ONO-1301 was set based on the most recent body weight (once a week), and ONO-1301 was filled in a capsule and administered by oral gavage. An empty capsule was administered to the control group in the same manner.

### (3) Confirmation of operating state of pacemaker

Electrocardiography (Lead II) was performed once a week during the period of induction of pathological condition (4 weeks). After administration of the test substance, electrocardiography was performed at the time of echocardiographic measurement. Examination with a stethoscope was performed every day. Specifically, the number of beats per 10 seconds was measured every day, and if an abnormality was found in examination with a stethoscope, an electrocardiogram was measured to confirm the presence or absence of pacing errors.

### 2) Symptom observation

The general condition of the animals (death, posture, activity, color of gums, the presence or absence of ascites, feces, etc.) and food intake were observed every day (at the time of administration in the morning and in the evening, i.e., twice). In the test, the animals were observed for death, and the survival rate in each administration group was calculated. The survival rate was observed twice daily for 6 months from the day of the start of administration of the test substance. Moribund animals were determined to be dead when they had no reaction in at least one item of examination of pinna, auditory, and nociception reflexes.

### 3) Measurement of body weight

The body weight was measured on the date of implantation of the pacemaker, the date of operation of the pacemaker, and every week thereafter.

### 4) Echocardiographic measurement

Echocardiographic measurement was performed before the preparation of the model (Pre); Week 4 (at the time of grouping) (before administration of the test substance); and 2 weeks, 4 weeks (1 month), 2 months, 3 months, 4 months, 5 months, and 6 months (at the time of dissection) after the start of administration of the test substance, as measurement points.

The echocardiographic measurement was performed without anesthesia, using an ultrasound imaging apparatus. A sector probe (10 MHz) was placed on the chest, and the diastolic left ventricular internal dimension (LVIDd), the systolic left ventricular internal dimension (LVIDs), the interventricular septal thickness at end-diastole (IVSTd), and the left ventricular posterior wall thickness in end diastole (LVPWd) were measured at M-mode. Moreover, the ejection fraction [EF=(LVIDd³-LVIDs³)/LVIDd³] and the fractional shortening [%FS=(LVIDd-LVIDs)×100/LVIDd] were calculated.

### 5) Results

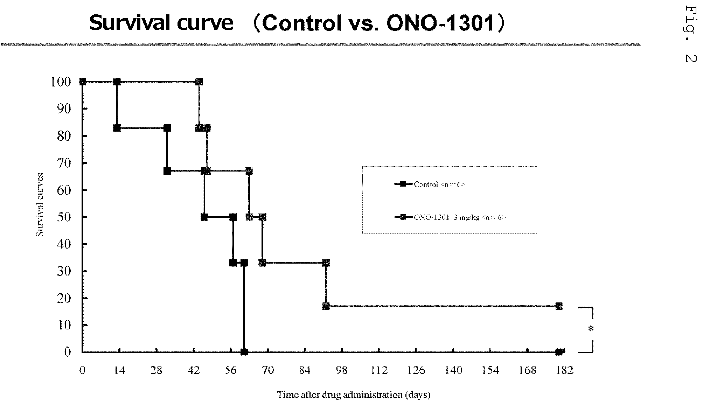

### (1) Survival rate (Fig. 2)

In the control group, death was observed from 13 days after the start of administration, and all animals (6/6) were dead by 61 days after the start of administration.

The survival rate 26 weeks after the start of administration was 0% (number of animals alive: 0/6). In contrast, death was first observed 44 days after the start of administration in the ONO-1301 repeated oral administration group. Although four deaths were observed by 92 days after the start of administration, one animal was alive 26 weeks after the start of administration (number of animals alive: 1/6).

In a Kaplan-Meier curve, a significant prolonging effect on the survival rate (*: P < 0.05) was observed in the ONO-1301 repeated oral administration group, compared with the control group.

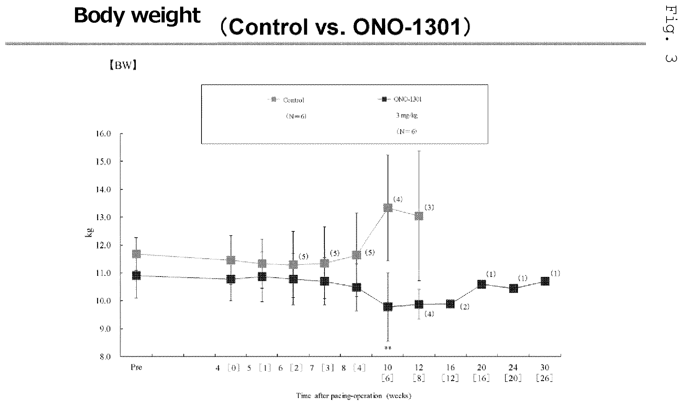

### (2) Changes in body weight (Fig. 3)

Body weight gain was suppressed in the ONO-1301 administration group, compared with the control group, 6 weeks and 8 weeks after the start of administration. Since body weight gain in the control group 6 weeks and 8 weeks after the start of administration was attributed to ascites, it was confirmed that administration of ONO-1301 suppresses ascites.

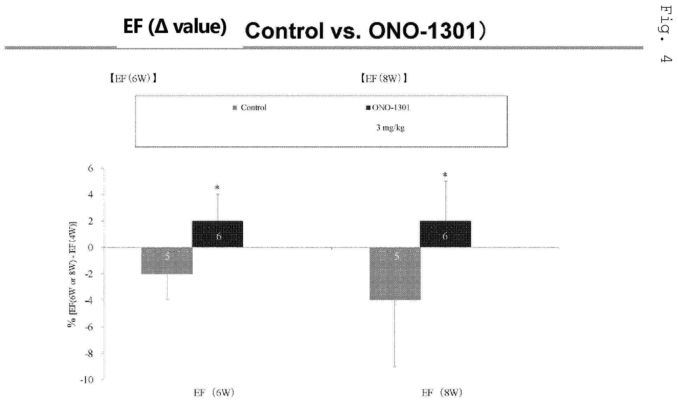

### (3) Cardiac function test (EF) (Fig. 4)

The EF before the preparation of the model was 72±5% (N=6) in the control group. The EF 6 weeks after the preparation of the model (2 weeks after the start of administration) was 43±3% (N=5), and the EF 8 weeks after the preparation of the model (4 weeks after the start of administration) was 41±5% (N=5), indicating a decrease in EF due to pathological condition. In contrast, the amount of change (Δ) in the EF was significantly higher in the ONO-1301 administration group, than in the control group, 6 weeks after the preparation of the model (2 weeks after the start of administration) and 8 weeks after the preparation of the model (4 weeks after the start of administration) (P < 0.05 vs. Control group in each).

### (4) Heart rate (echocardiography)

Oral administration of ONO-1301 at 3 mg/kg provided no effect on the heart rate in echocardiography at Tₘₐₓ (during from 1.5 to 2.5 hours after oral administration). This result confirmed that no antihypertensive action associated with vasodilator action was exhibited at the above dose at Tₘₐₓ (Cₘₐₓ).

In the group of repeated oral administration of ONO-1301 at 3 mg/kg twice per day, death was first observed 44 days after the start of administration, and one animal was alive 26 weeks after the start of administration (at the time of final evaluation 30 weeks after the preparation of the model) (number of animals alive: 1/6). There was no problem in the operating conditions of the pacemaker in the animals. Moreover, the time of death due to heart failure was prolonged compared with the control group, and a significant prolonging effect on the survival rate was thus observed. Further, a significant improvement effect on left ventricular systolic dysfunction during heart failure was observed in the EF value in the ONO-1301 repeated oral administration 2 weeks and 4 weeks after the start of administration, compared with the control group. Suppression of body weight gain due to ascites was also observed; however, it was not significant.

In the long-term administration to the canine model of rapid pacing-induced severe dilated cardiomyopathy, it was revealed from the Kaplan-Meier curve that the ONO-1301 repeated oral administration group showed significant extension of the survival rate and a significant improvement effect on left ventricular systolic dysfunction during heart failure, compared with the control group.

It was confirmed that early therapeutic intervention by repeated oral administration of ONO-1301 suppresses deterioration of cardiac function in dilated cardiomyopathy and extends the survival rate, thereby preventing aggravation of heart failure.

### 5. Production of PLGA·MS long-acting preparations

### 1) Poly(lactic-co-glycolic acid) (PLGA) microsphere (MS) preparations of three compounds, i.e., ozagrel hydrochloride, camostat mesilate, and sildenafil citrate were prepared.

### 2) Production Method

1.0 g of one of the above compounds and 4.0 g of PLGA 5-50 (produced by Mitsui Chemicals, Inc.) were dissolved in a mixture of 40 mL of dichloromethane and 16 mL of methanol. A 0.5% aqueous PVA solution (polyvinyl alcohol; produced by Nippon Synthetic Chemical Industry Co., Ltd.) and a solution of the compound were fed to a homomixer (produced by Primix Corporation) at a rate of 400 mL/min and 2 mL/min, respectively; and the homomixer was stirred at 3500 rpm. Subsequently, after 3 hours of in-water drying, 2 hours of standing, discarding of 1/4 of the supernatant, and 1 day of standing under refrigeration overnight, the particles were washed by centrifugation (Milli-Q water, twice), and collected. The collected particles were freeze-dried. The freeze-dried particles were subjected to UV absorption measurement to determine the concentration of the compound contained in the particles. The particle morphology and the average particle size were confirmed by optical microscopy observation. Further, the sustained release of PLGA·MS comprising PLGA 5-50 (lactic acid/glycolic acid = 50/50, molecular weight: 50,000) was confirmed to be about 4 weeks.

### 3) Production results

### (1) C·MS; PLGA·MS of Ozagrel Hydrochloride (Preparation 1)

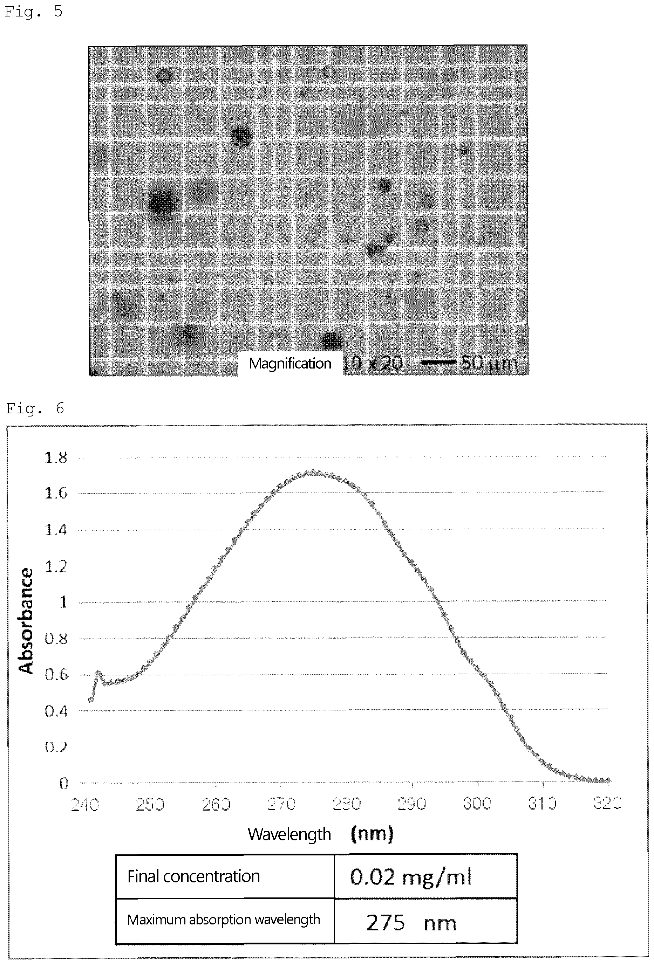

Particles with a total particle weight of 3.31 g, a compound content of 0.24 g, and an inclusion rate of 7.4%, as shown in Table 18, were obtained by the above production method. Fig. 6 shows the UV absorption spectrum at the time of measuring the inclusion rate (content). As shown in Fig. 5, the optical microscopy observation results confirmed that the particles have an average particle size of about 30 µm.

**Table 32**

| Amount fed (g) | Total weight (g) | Amount of compound contained (g) | Inclusion rate (%) | Appearance | Collected particles (%) |
|---|---|---|---|---|---|
| 5.00 | 3.31 | 0.24 | 7.4 | White powder | 66.2 |

### (2) B·MS; PLGA·MS of Camostat Mesylate (Preparation 2)

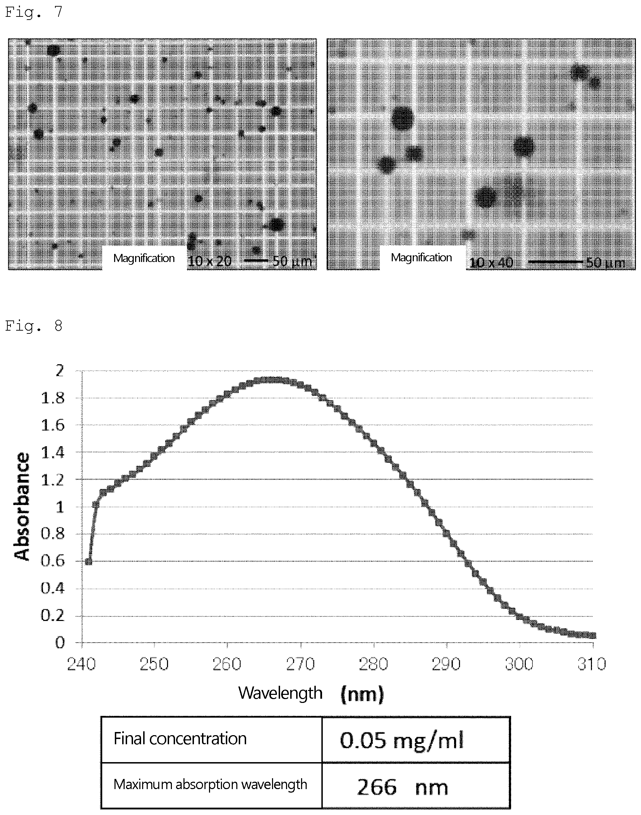

Particles with a total particle weight of 3.47 g, a compound content of 0.20 g, and an inclusion rate of 5.8%, as shown in Table 19, were obtained by the above production method. Fig. 8 shows the UV absorption spectrum at the time of measuring the inclusion rate (content). As shown in Fig. 7, the optical microscopy observation results confirmed that the particles have an average particle size of about 30 µm.

**Table 33**

| Amount fed (g) | Total weight (g) | Amount of compound contained (g) | Inclusion rate (%) | Appearance | Collected particles (%) |
|---|---|---|---|---|---|
| 5.00 | 3.47 | 0.20 | 5.8 | White powder | 69.4 |

### (3) I·MS; PLGA·MS of Sildenafil Citrate (Preparation 3)

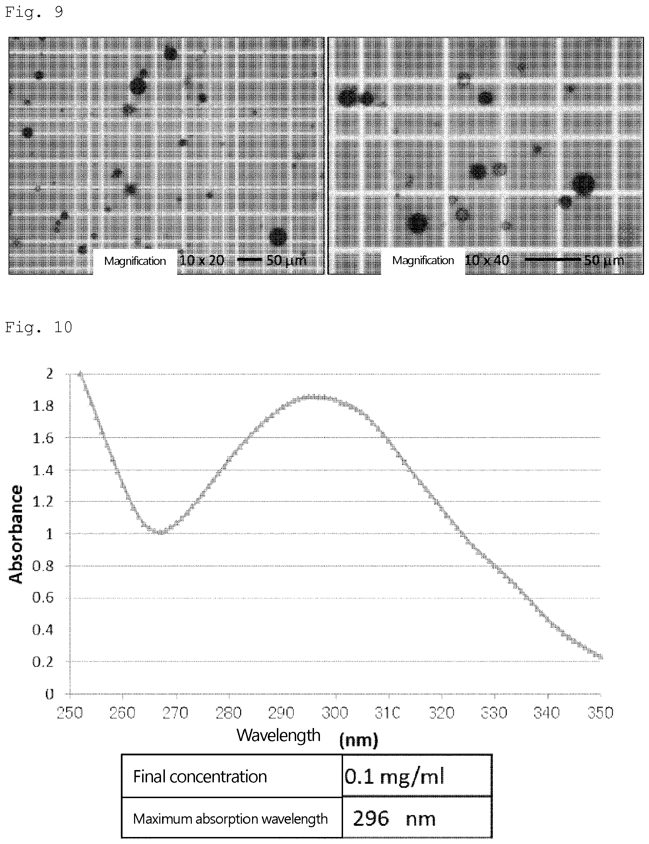

Particles with a total particle weight of 3.98 g, a compound content of 0.45 g, and an inclusion rate of 11.2%, as shown in Table 20, were obtained by the above production method. Fig. 9 shows the UV absorption spectrum at the time of measuring the inclusion rate (content). As shown in Fig. 10, the optical microscopy observation results confirmed that the particles have an average particle size of about 30 µm.

**Table 34**

| Amount fed (g) | Total weight (g) | Compound contained (g) | Inclusion rate (%) | Appearance | Collected particles (%) |
|---|---|---|---|---|---|
| 5.00 | 3.98 | 0.45 | 11.2 | White powder | 79.6 |

## Claims

1. A pharmaceutical composition for use in preventing and/or treating an intractable heart tissue fibrosis disease accompanied by chronic heart failure.

2. The pharmaceutical composition according to claim 1, comprising a protease inhibitor.

3. The pharmaceutical composition according to claim 1, comprising a thromboxane A₂ synthase inhibitor and/or a thromboxane A₂ synthase antagonist.

4. The pharmaceutical composition according to claim 1, comprising a phosphodiesterase (PDE) inhibitor.

5. The pharmaceutical composition according to claim 1, comprising a tyrosine kinase inhibitor.

6. The pharmaceutical composition according to claim 1, comprising an HMG-CoA reductase inhibitor.

7. The pharmaceutical composition according to claim 1, comprising an antifibrotic agent.

8. The pharmaceutical composition according to claim 1, comprising at least two members selected from the group consisting of a protease inhibitor, a thromboxane A₂ synthase inhibitor, a thromboxane A₂ synthase antagonist, a phosphodiesterase (PDE) inhibitor, a tyrosine kinase inhibitor, an HMG-CoA reductase inhibitor, and an antifibrotic agent.

9. The pharmaceutical composition according to any one of claims 1 to 8, comprising at least one member selected from the group consisting of the following compounds (1) to (6) and salts thereof:
(1) camostat as a protease inhibitor;
(2) ozagrel as a thromboxane A₂ synthase inhibitor;
(3) theophylline, cilostazol, and sildenafil as phosphodiesterase inhibitors;
(4) nintedanib as a tyrosine kinase inhibitor;
(5) lovastatin as an HMG-CoA reductase inhibitor; and
(6) pirfenidone as an antifibrotic agent.

10. The pharmaceutical composition according to any one of claims 1 to 9, which is a long-acting preparation further comprising a biodegradable polymer.

11. The pharmaceutical composition according to claim 10, wherein the long-acting preparation is a microsphere preparation, a microcapsule preparation, or a nanosphere preparation.

12. The pharmaceutical composition according to claim 10, wherein the biodegradable polymer is a poly(lactic-co-glycolic acid), and the long-acting preparation is a microsphere preparation.

13. The pharmaceutical composition according to claim 11, which comprises at least one member selected from the group consisting of the following compounds (1) to (5) and salts thereof:
(1) camostat as a protease inhibitor;
(2) ozagrel as a thromboxane A₂ synthase inhibitor;
(3) cilostazol and sildenafil as phosphodiesterase inhibitors;
(4) nintedanib as a tyrosine kinase inhibitor; and
(5) pirfenidone as an antifibrotic agent.

14. The pharmaceutical composition according to any one of claims 1 to 13, which is for oral administration, intravenous administration, intracoronary administration, inhalation, intramuscular injection, subcutaneous administration, transmucosal administration, transdermal administration, or cardiac patch application.

15. The pharmaceutical composition according to any one of claims 1 to 14, wherein the intractable heart tissue fibrosis disease accompanied by chronic heart failure is dilated cardiomyopathy, ischemic cardiomyopathy, myocardial infarction, angina pectoris, arteriosclerosis, vasculitis syndrome, myocarditis, hypertrophic cardiomyopathy, aortic valve stenosis, valvular disease, aortic regurgitation, HFpEF (heart failure with preserved ejection fraction), diastolic dysfunction, contractile dysfunction, supraventricular tachyarrhythmia, congestive heart failure, coronary artery disease, idiopathic cardiomyopathy, or atrial fibrillation.
